# FASCICULE DE BREVET EUROPEEN

(11) **EP 3 571 311 B1**
(45) Date de publication et mention de la délivrance du brevet: **02.12.2020**
(21) Numéro de dépôt: 17801075.7
(22) Date de dépôt: 27.10.2017
(51) Int. Cl.: C12Q 1/04

(54) **MILIEU DE CULTURE SELECTIF POLYVALENT POUR L'ISOLEMENT ET LA SELECTION DE BACTERIES RESISTANTES A LA COLISTINE ET A LA VANCOMYCINE**
SELEKTIVES MULTIFUNKTIONELLES KULTURMEDIUM ZUR ISOLIERUNG UND AUSWAHL VON COLISTIN-RESISTENTEN UND VANCOMYCIN-RESISTENTEN BAKTERIEN
SELECTIVE MULTIPURPOSE CULTURE MEDIUM FOR ISOLATING AND SELECTING COLISTIN-RESISTANT AND VANCOMYCIN-RESISTANT BACTERIA

(30) Priorité: 18.01.2017 FR 1750383
(43) Date de publication de la demande: 27.11.2019
(73) Titulaire: Fondation Méditerranée Infection, 13005 Marseille (FR); Université d'Aix Marseille, 13284 Marseille Cedex 07 (FR); Assistance Publique - Hôpitaux de Marseille, 13005 Marseille (FR); Centre National de la Recherche Scientifique (CNRS), 75016 Paris (FR); INSERM (Institut National de la Santé et de la Recherche Médicale), 75013 Paris (FR)
(72) Inventeur: ROLAIN, Jean-Marc, 13006 Marseille (FR); LE PAGE, Stéphanie, 69002 Lyon (FR); BARDET, Lucie, 83110 Sanary-sur-Mer (FR)
(74) Mandataire: Cabinet Beau de Loménie
(86) Numéro de dépôt international: PCT/FR2017/052967
(87) Numéro de publication internationale: WO 2018/134487

(56) Documents cités:
- NORDMANN PATRICE ET AL: "A Universal Culture Medium for Screening Polymyxin-Resistant Gram-Negative Isolates", JOURNAL OF CLINICAL MICROBIO, AMERICAN SOCIETY FOR MICROBIOLOGY, UNITED STATES, vol. 54, no. 5, 1 mai 2016 (2016-05-01), pages 1395-1399, XP009190074, ISSN: 1098-660X cité dans la demande
- GIANCARLO CECCARELLI ET AL: "The role of vancomycin in addition with colistin and meropenem against colistin-sensitive multidrug resistant Acinetobacter baumannii causing severe infections in a Paediatric Intensive Care Unit", BMC INFECTIOUS DISEASES, vol. 15, no. 1, 30 septembre 2015 (2015-09-30), XP055404064, DOI: 10.1186/s12879-015-1133-3
- PATRICE NORDMANN ET AL: "Rapid Detection of Polymyxin Resistance in Enterobacteriaceae", EMERGING INFECTIOUS DISEASES, vol. 22, no. 6, 1 juin 2016 (2016-06-01), pages 1038-1043, XP055404046, US ISSN: 1080-6040, DOI: 10.3201/eid2206.151840 cité dans la demande
- Jvo Siegrist ET AL: "Legionnaires' Disease A Potentially Fatal Form of Pneumonia", , 1 janvier 2012 (2012-01-01), XP055404117, Extrait de l'Internet: URL:https://www.sigmaaldrich.com/content/d am/sigma-aldrich/docs/Sigma-Aldrich/Brochu re/1/mibi-focus-4-2.pdf

## Description

La présente invention concerne un nouveau milieu de culture permettant la croissance de bactéries multi-résistantes de fort intérêt clinique. Il est en effet capable d'isoler et de sélectionner les bactéries à Gram-négatif résistantes à la colistine telles que des entérobactéries ainsi que les bactéries à Gram-positif résistantes à la vancomycine telles que des entérocoques, à partir d'échantillons cliniques ou de souches bactériennes. Ce milieu présente également la propriété de pouvoir isoler des souches bactériennes à Gram-négatif habituellement présentes dans les prélèvements pulmonaires des patients atteints par la mucoviscidose.

Les infections causées par des entérobactéries multi-résistantes et les Entérocoques résistants à la vancomycine représentent un problème majeur de santé publique à travers le monde.

Les Entérobactéries (à savoir la famille des Enterobacteriaceae) sont fréquemment rencontrées en pathologie infectieuse. Elles sont les hôtes de l'homme et des animaux chez lesquels elles résident principalement au niveau de l'intestin. On peut cependant les retrouver dans la cavité buccale, les régions humides de la peau en particulier le périnée, les fosses nasales et les voies génitales féminines dans lesquelles elles peuvent constituer une flore transitaire. L'espèce Escherichia coli y joue un rôle prépondérant en raison de sa présence constante et de sa large prédominance sur les autres espèces. D'autres espèces comprennent des Entérobactéries pathogènes des genres *Proteus* et *Klebsiella* ainsi que *Citrobacter, Hafnia, Providencia, Enterobacter, Morganella,* et *Yersinia.*

Klebsiella pneumoniae et Escherichia coli à elles deux représentent 90% des infections par des entérobactéries.

L'émergence mondiale d'Entérobactéries multirésistantes, en particulier des bactéries productrices de carbapénèmases, a conduit à la réutilisation de la colistine en tant que traitement antibiotique de dernier recours (1-3) contre les Entérobactéries pathogènes.

La colistine (ou polymyxine E), un produit obtenu par fermentation de *Paenibacillus po*/*ymyxa,* est constituée de composés peptidiques polycationiques à savoir la Colistine A de formule (Ia) laquelle comprend une chaine d'acide gras: (S)-6-methyloctanoyl et la colistine B de formule (Ib) ci-après laquelle comprend (chaine d'acide gras: 6-methylheptanoyl)(4). Il s'agit d'une classe d'antibiotique développée dans les années 1950. La Colistine est administrée soit par voie parentérale sous la forme d'une prodrogue inactive : le colistiméthate de sodium (CMS), soit par voie orale ou topique sous forme de sulfate de colistine.

Au cours des 10 dernières années, l'utilisation de la colistine s'est généralisée, entraînant l'apparition de résistances à la colistine (1, 3). Ce phénomène s'est particulièrement illustré chez les animaux en raison de l'utilisation intensive de la colistine dans les aliments vétérinaires dans de nombreux pays (2, 5, 6). On a constaté l'émergence de résistances acquises par mutation chromosomique spontanée vis à vis de la colistine. La colistine est un antibiotique qui possède un fort pouvoir d'induction spontanée de mutants résistants au sein d'une même population bactérienne.

Le mécanisme de résistance des souches intrinsèquement résistantes à la colistine est différent de celui de souches non intrinsèquement résistantes à la colistine mais résistantes à la colistine de façon acquise par mutation chromosomique spontanée (non transférable) ou par l'acquisition d'un plasmide de résistance (transférable de bactérie à bactérie). Les différents mécanismes conférant la résistance aux polymyxines, notamment à la colistine aboutissent à la modification du lipide A, un constituant du lipopolysaccharide (LPS), entraînant une réduction de l'affinité des polymyxines aux agents pathogènes (7). Jusqu'à récemment, tous les gènes de résistance à la colistine connus étaient d'origine chromosomique (8).

Liu et ses collègues ont rapporté le premier mécanisme de résistance à la colistine à médiation plasmidique, qu'ils ont appelé gène mcr-1 (6), suivi récemment par le gène mcr-2 et les variants de mcr-1 (9).

Ces gènes plasmidiques codent pour une phosphoéthanolamine transférase capable de modifier le lipide A en lui ajoutant une phosphoéthanolamine, réduisant ainsi les interactions électrostatiques entre la colistine et le LPS (6). L'étude originale a rapporté la forte capacité de transfert in vitro de mcr-1, suggérant que ce gène de résistance est susceptible de se propager rapidement parmi les principaux pathogènes humains (10-12). Cette découverte soulève ainsi des inquiétudes en raison de l'absence de mise sur le marché de nouveaux antibiotiques. La recherche du gène mcr-1 a été effectuée sur des souches dont le mécanisme de résistance était inconnu et un nombre important d'entre elles le possédait (13-14).

Il est important de détecter cette résistance à la colistine car le cas échéant les cliniciens doivent adapter le traitement et isoler le patient. Le développement d'une méthode efficace de détection précoce de ces souches résistantes à la colistine est ainsi devenu une priorité (15-17).

La majorité des bactéries possédant le gène mcr-1 ont une faible Concentration Minimale Inhibitrice (CMI) de colistine, de l'ordre de 4 - 8 µg/mL (5). Les bactéries intrinsèquement résistantes à la colistine ont une CMI supérieure à 16 µg/mL.

Actuellement, les milieux de culture pour sélectionner les bactéries résistantes à la colistine sont adaptés pour isoler des bactéries intrinsèquement résistantes (à l'exception du milieu Superpolymyxin), c'est à dire fortement résistantes à une CMI supérieure à 16 µg/mL et ils n'ont pas de caractère polyvalent, ils ne permettent pas de sélectionner les bactéries à Gram-positif résistantes à la vancomycine en particulier.

En outre, les milieux sélectifs pour Entérobactéries comprennent du lactose afin de les sélectionner sélectivement par rapport aux autres bactéries à Gram-négatif (non fermentantes).

Les milieux de culture commerciaux disponibles contenant des polymyxines ne permettent pas l'isolement de bactéries faiblement résistantes à résistance acquise du type mcr-1 (voir Tableau 1 ci-après en annexe), excepté un nouveau milieu de culture sélectif pour l'isolement des bactéries à Gram-négatif résistantes aux polymyxines, nommé SuperPolymyxin, comprenant une gélose EMB (éosine et bleu de méthylène) et contenant 3,5 µg/mL de colistine, 10 µg/mL de daptomycine et 5 µg/mL d'amphotéricine B et 10 g/ de lactose(23) (voir Tableau 1 ci-après en annexe). Ceccarelli et al (2015. BMC Infect Dis, 15, 393) décrit un milieu de culture préparé avec Mueller Hinton broth dans lequel vancomycine et colistin sont ajoutés.

La quasi-totalité des bactéries à Gram-positif sont résistantes à la colistine. En conséquence, pour sélectionner sélectivement les bactéries à Gram-négatif résistantes à la colistine notamment ayant le gène de résistance mcr-1, le milieu de culture SuperPolymyxin comprend un antibiotique anti-Gram-positif, à savoir la daptomycine (18).

Un inconvénient du milieu Superpolymyxin est qu'il ne permet pas d'isoler et sélectionner les bactéries à Gram-positif résistantes à la vancomycine du fait que la daptomycine inhibe la plupart des bactéries résistantes à la vancomycine.

En outre, si on remplace la daptomycine par la vancomycine dans le milieu SuperPolymyxin, les bactéries *Escherichia coli* possèdant le gène mcr-1 ne poussent plus. Le milieu perd donc la capacité de sélection des principales bactéries résistantes à la colistine que sont les *E coli* ce qui explique le choix de la daptomycine dans le milieu Superpolymyxin. Les auteurs Nordmann et al (18) suggèrent que la combinaison de colistine et vancomycine inhibe la sélection de bactéries à Gram-négatif.

Un premier but de la présente invention est de fournir un milieu de culture amélioré de sensibilité suffisante pour isoler non seulement les bactéries à Gram-négatif, notamment les Entérobactéries, fortement et intrinsèquement résistantes à la colistine mais également les bactéries plus faiblement résistantes à résistance acquise à la colistine.

Un autre but de la présente invention est également de fournir un milieu de culture apte à détecter en outre des bactéries à Gram-positif résistantes à la vancomycine, notamment les Entérocoques sans nuire à l'aptitude du milieu à détecter les bactéries résistantes à la colistine. Il est important de détecter ces deux résistances car la colistine et la vancomycine sont des antibiotiques de dernier recours et si des souches résistantes à ces deux antibiotiques se propagent dans un hôpital on risque de ne plus pouvoir traiter les patients. Il est donc primordial de détecter ces bactéries afin de pouvoir isoler les patients porteurs.

Les milieux sélectifs pour les bactéries à Gram-positif sont en général des géloses enrichies au sang sans chromogène sans sucre et additionné d'antibiotique anti Gram-négatif tels que le milieu étudié ci-après :CNA (colistine-acide nalidixique)/esculine).

Un autre inconvénient du milieu Superpolymyxin est qu'il ne permet pas d'isoler et de sélectionner efficacement certaines bactéries à Gram négatif présentes dans les prélèvements pulmonaires de patients atteints de mucoviscidose (voir tableau E ci-après).

Un autre but de la présente invention est de fournir un milieu de culture sélectif qui permet d'isoler les bactéries retrouvées dans les prélèvements pulmonaires de patients atteints de mucoviscidose de façon plus performante.

D'autre part, certaines des souches naturellement résistantes à la colistine testées comme contrôle ont été inhibées sur le milieu SuperPolymyxin, notamment des souches de Morganella morganii et Providencia alcalifaciens.

Un autre inconvénient du milieu Superpolymyxin est qu'il ne permet de bien visualiser les colonies car celles-ci apparaissent en rose sur fond rouge du fait du chromogène EMB.

Un autre but de la présente invention est de fournir un milieu de culture qui fournisse une meilleure visibilité des colonies de bactéries après culture, et de surcroit une visibilité différentiée entre les bactéries à Gram-positif et bactéries à Gram-négatif.

Dans Nordmann et al (18) les auteurs n'ont pas démontré la capacité de sélection sur les dites bactéries par culture d'échantillons biologiques directement sans préparation préalable des bactéries et notamment des échantillons de selles.

Un autre but de la présente invention est de fournir un milieu qui permettent d'isoler et de sélectionner les dites bactéries par culture d'échantillons biologiques directement sans préparation préalable des bactéries et notamment des échantillons de selles pour la sélection des Entérobactéries résistantes à la colistine et des Entérocoques résistants à la vancomycine.

Pour ce faire, la présente invention fournit un milieu de culture solide sélectif polyvalent pour isoler et sélectionner, à partir d'échantillons en contenant, des bactéries choisies parmi (a) les bactéries à Gram-négatif fermentantes et non fermentantes résistantes à la colistine et (b) les bactéries à Gram-positif résistantes à la vancomycine, caractérisé en ce qu'il est constitué essentiellement d'une gélose d'agar additionnée d'un agent chromogène consistant dans du pourpre de bromocrésol, un sucre et de deux composés antibiotiques consistant dans la vancomycine et la colistine.

La présence de vancomycine permet d'éviter la contamination des cultures par des souches à Gram-positif présentes dans des échantillons cliniques et confère aussi une 2ième sélectivité très intéressante au milieu à savoir l'isolement des bactéries résistantes à la vancomycine.

On a découvert de façon surprenante et non élucidée que la mise en œuvre d'une combinaison de colistine et vancomycine dans un milieu de culture permet la double sélectivité revendiquée avec les propriétés avantageuses conformes à tous les buts de l'invention sous réserve de mettre en œuvre un agent chromogène spécifique à savoir l'agent chromogène le Pourpre de Bromocrésol. En particulier, avec les autres chromogènes et cette combinaison d'antibiotiques, certaines des bactéries sensibles à la colistine ou à la vancomycine concernées poussaient et/ou certaines bactéries résistantes à la colistine ou à la vancomycine concernées étaient inhibées.

Le pourpre de bromocrésol, ou bromocrésol pourpre, ou violet de bromocrésol, ou « purple » nommé plus complètement « 5',5"-dibromo-o-crésolsulfonephthaléine », est un colorant de la famille des sulfonephtaléines.

De préférence, dans le milieu selon l'invention, le dit sucre est du glucose.

L'agent chromogène pourpre de bromocrésol combiné au glucose dans de l'agar permet en outre de mieux identifier les colonies de bactéries à Gram négatif aussi bien les bactéries fermentantes (Entérobactéries) que les bactéries non fermentantes ainsi que les bactéries à Gram-positif par une couleur contrastée jaune sur fond violet plus contrastée dans tous les cas.

En outre, le glucose comme substrat glucidique de fermentation permet une différenciation de l'aspect des colonies des bactéries à Gram-négatif, notamment d'Entérobactéries, celles-ci étant de tailles plus importantes, à savoir de 2-3mm, que les colonies d'Entérocoques de tailles plus petites à savoir de 0.1-1mm.

Plus particulièrement, un milieu de culture selon l'invention comprend dans de l'eau des concentrations de:
- 2 à 10% en poids en agar contenant de 0.01 à 0.1% de dit agent chromogène pourpre de bromocrésol,
- 0.1 à 10% en poids de sucre, de préférence de glucose,
- 2 à 8 µg/mL de sulfate de colistine, et
- 25 à 100 µg/mL de vancomycine.

Plus particulièrement encore, un milieu de culture selon l'invention comprend :
- 3.1% en poids en agar contenant de 0.065% de bromocrésol pourpre,
- 0.75% de glucose,
- 4.µg/mL de sulfate de colistine, et
- 50.µg/mL de vancomycine.

La présente invention fournit également une méthode d'isolement et de sélection de bactéries choisies parmi (a) les bactéries à Gram-négatif résistantes à la colistine, (b) les bactéries à Gram-positif résistantes à la vancomycine, caractérisé en ce qu'on réalise une culture d'un d'échantillon biologique en contenant, de préférence un échantillon de prélèvement clinique, à l'aide d'un milieu de culture sélectif polyvalent solide selon l'invention.

Dans un premier mode de réalisation, les dites bactéries à Gram-négatif résistantes à la colistine sont des Entérobactéries.

Plus particulièrement, les dites entérobactéries pathogènes sont choisies parmi les bactéries suivantes *Escherichia, Shigella, Salmonella, Klebsiella, Enterobacter, Providencia, Morganella, Citrobacter, Hafnia* et *Yersinia.*

Plus particulièrement encore, les dites bactéries à Gram-négatif résistantes à la colistine comprennent *Escherichia coli, Klebsiella pneumoniae, Klebsiella oxytoca, Enterobacter aerogenes, Enterobacter asburiae, Enterobacter cloacae, Salmonella enterica, Proteus mirabilis, Proteus vulgaris, Providencia alcalifaciens, Providencia rettgeri, Morganella morganii et Serratia marcescens.*

Plus particulièrement encore, les dites bactéries à Gram-négatif résistantes à la colistine comprennent Escherichia coli.

Dans un autre mode de réalisation, les dites bactéries à Gram-négatif résistantes à la colistine sont des bactéries présentes dans les prélèvements cliniques, de préférence des prélèvements pulmonaires, de patients atteints par la mucoviscidose.

Plus particulièrement, les dites bactéries présentes dans les prélèvements cliniques des patients atteints par la mucoviscidose sont choisies parmi :
- les bactéries naturellement résistantes à la colistine suivantes : *Burkholderia cepacia, Chryseobacterium gleum, Chryseobacterium oranimense, Chryseobacterium indologenes, Inquilinus limosus, Pandoraea pulmonicola, Brevundimonas diminuta, Shewanella xianemensis, Shewanella putrefaciens* et *Ochrobactrum anthropi* ; et
- les bactéries ayant une résistance acquise à la colistine suivantes : *Acinetobacter pitti, Acinetobacter nosocomialis, Achromobacter xylosoxidans, Stenotrophomonas maltophilia, Pseudomonas aeruginosa* et *Acinetobacter baumannii.*

Plus particulièrement encore, les dites bactéries à Gram-négatif résistantes à la colistine sont choisies parmi (i1) des bactéries naturellement résistantes à la colistine, (i2) des bactéries résistantes à la colistine de façon acquise par mutation chromosomique spontanée et (i3) des bactéries résistantes à la colistine par l'acquisition d'un plasmide de résistance contenant le gène mcr-1 ou un autre gène pouvant conférer la résistance à la colistine.

Plus particulièrement encore, les dites bactéries à Gram-négatif résistantes à la colistine sont choisies parmi des bactéries résistantes à la Colistine par l'acquisition d'un plasmide de résistance contenant le gène mcr-1.

Dans un autre mode de réalisation, les dites bactéries sont des bactéries à Gram-positif résistantes à la vancomycine choisies parmi :
- les bactéries ayant une résistance acquise à la vancomycine des genres Staphylococcus et Enterococcus, et
- les bactéries naturellement résistantes à la vancomycine des genres *Pediococcus, Weissela, Lactobacillus* et *Leuconostoc.*

Plus particulièrement encore, les dites bactéries à Gram positif résistantes à la vancomycine sont choisies parmi les bactéries des genres suivants : *Staphylococcus aureus, Enterococcus faecium, Enterococcus faecalis, Enterococcus gallinarum, Leuconostoc lactis, Weissela cibaria* et *Weissela confusa.*

Plus particulièrement encore, les dites bactéries à Gram-positif résistantes à la vancomycine sont des Entérocoques.

Plus particulièrement encore, on réalise une culture d'un échantillon de prélèvement clinique, de préférence de selles.

Plus particulièrement encore, un procédé selon l'invention comprend les étapes suivantes consistant à:
a) inoculer ledit milieu de culture avec un échantillon comprenant des dites bactéries;
b) incuber ledit milieu de culture inoculé dans des conditions appropriées pour la croissance des dites bactéries présentant une dite résistance à la colistine ou à la vancomycine, de préférence pendant 24 heures à 48 heures, de préférence de 24 à 36 heures, et
c) détecter les colonies formées sur ledit milieu inoculé et cultivé, lesdites colonies contenant lesdites bactéries qui présentent une dite résistance.

Selon la présente invention, il a été démontré que le milieu selon l'invention est capable d'isoler toutes les souches à Gram-négatif résistantes à la colistine que ce soit de haut niveau (CMI élevée) ou de bas niveau (CMI faible) et ayant une résistance intrinsèque ou acquise et avec une sensibilité de 100%.

Le milieu selon l'invention peut être utilisé comme outil de dépistage sur des échantillons de selles positifs en PCR pour le gène mcr-1, et permet ainsi l'isolement de nouvelles souches résistantes porteuses du gène mcr-1. Cet isolement est facilité sur le milieu selon l'invention, en raison de la couleur jaune des colonies correspondant aux Entérobactéries recherchées.

La culture sur le milieu selon l'invention de ces échantillons permet d'isoler des bactéries à Gram-positif naturellement résistantes à la vancomycine telles que Pediococcus, Lactobacillus, Weissela, mais également des Entérocoques résistants à la vancomycine qui constituent des bactéries d'intérêt clinique. Le milieu selon l'invention a également montré une sensibilité de 100% pour l'isolement de ces bactéries, reconnaissables par leurs colonies, de couleur contrastée avec le glucose (jaune sur fond violet) également, mais plus petites (0.1-1mm) que les entérobactéries (2-3mm).

Sur le milieu SuperPolymyxin, seules les E. coli sont efficacement différenciables avec un reflet métallique, toutes les autres souches sont rouges/rosées sur gélose rouge.

Ce milieu de culture permet l'isolement des bactéries dans les prélèvements pulmonaires des patients atteints de mucoviscidose d'efficacité comparable au milieu Cepacia de référence pour ces bactéries.

Enfin, en termes de couts, la comparaison des prix des géloses est inférieure pour le milieu selon l'invention par rapport aux milieux SuperPolymyxin et Cepacia.

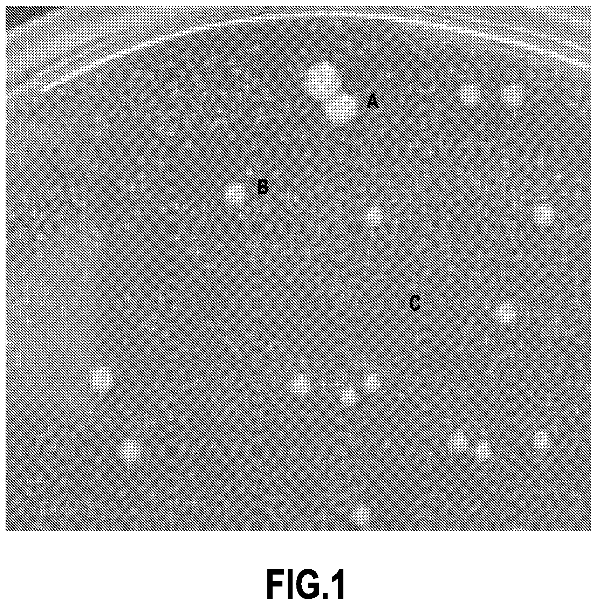

D'autres caractéristiques et avantages de la présente invention apparaitront à la lumière de la description détaillée qui va suivre faite en référence à la figure 1 qui illustre les aspects des colonies obtenues par culture d'un échantillon clinique de selle (écouvillon rectal) sur milieu LBJMR selon l'invention pour les bactéries A= *E coli*; B= *K. pneumoniae* et C= E. faecium. Les espèces des colonies ayant différents aspects A (2 grosses colonies d' *E coli*) et B (15 colonies de tailles moyennes de K. pneumoniae) sont résistantes à la colistine (CMI de 2 et 64 µg/mL, respectivement), et C (toutes les petites colonies d' .*E faecium*) est résistante à la vancomycine avec une CMI>256 µg/mL.

### 1- Matériels et méthodes

### 1-1 Souches bactériennes et échantillons

Un ensemble de 102 souches d'Entérobactéries a été utilisé, parmi lesquelles 76 souches possèdent une résistance acquise à la colistine (1, 19-21) dont 33 souches avec le gène mcr-1 (22-25), 6 souches sont naturellement résistantes et 20 souches sont sensibles à la colistine (Tableau 2 ci-après en annexe). Cet ensemble de 102 souches comprend 31 souches de E. coli, 35 souches de Klebsiella pneumoniae. Les gènes de résistance connus éventuellement impliqués ont été séquencés comme décrit précédemment (1). La détection par PCR du gène mcr-1 a été réalisée comme décrit par Chabou et al. (15).

Cet ensemble comprenait également 26 souches de bactéries à Gram-positif, incluant 8 Entérocoques résistants à la vancomycine (ERV) (26), 3 genres intrinsèquement résistants et 15 souches sensibles (27) (Tableau 2 ci-après en annexe). Les gènes de résistance à la vancomycine vanA et vanB ont été recherchés par PCR comme précédemment décrit sur les souches résistantes (28).

Un ensemble de 1073 échantillons de selles a été utilisé, dont 899 d'origine humaine et provenant de différentes régions du monde : Marseille, France (212), Angers, France (128), Thaïlande (212), Laos (189), Nigeria (143), Sénégal (15). Les autres échantillons étaient d'origine animale : poulets d'Algérie (10), rongeurs du Cambodge (21) et du Laos (60), cochons (16) et chèvres (46) du Laos ; et quelques-uns (21) issus de l'environnement du Laos (eau, sol).

### 1-2 Tests de sensibilité aux antibiotiques

Les CMI de la colistine et de la vancomycine ont été déterminées par la méthode des E-test (bioMérieux, Marcy-l'Étoile, France) et évaluées en suivant les recommandations du système expert de l'EUCAST (http://www/eucast.org). Les CMI de la Daptomycine ont également été déterminées par cette méthode pour les 4 souches de référence Enterococcus faecium (DSM 17050, 25698, 25697 et 13590). Des colonies fraîches ont été repiquées sur le milieu Trypticase Soy Agar (TSA, Beckton-Dickinson, Heidelberg, Allemagne) puis mises en suspension dans l'eau stérile afin d'obtenir une densité optique égale à 0,5 MacFarland (1,5X108 UFC/mL) comme recommandé par l'EUCAST. Dans les premières expérimentations concernant la mise au point du milieu de culture, 10 microlitres de la solution ont été directement étalés avec une öse sur les différents milieux afin d'obtenir un inoculum bactérien de 106 UFC. Afin d'évaluer les limites de détection de chaque bactérie, des dilutions au dixième en cascade ont été effectuées dans du tampon phosphate (PBS), et 10 µL de chaque dilution ont été déposés sur les géloses. La concentration bactérienne viable a été calculée par comptage de colonies en les étalant en parallèle sur une gélose TSA pour contrôle. Ces expérimentations ont été menées en duplicat.

La croissance ou l'inhibition de ces bactéries a été observée après 24H d'incubation à 37°C.

### 1-3 Développement du milieu LBJMR selon l'invention

### 1-3-1 Choix de la base d'agar

Afin de choisir le milieu le plus adapté à la sélection d'Entérobactéries résistantes à la colistine, dont les souches possédant le gène mcr-1, 8 espèces représentatives ont été cultivées à la quantité de 10⁶ UFC sur cinq milieux de culture additionnés de différentes concentrations de sulfate de colistine (MP Biomedicasl, Illkirch, France), allant de 0 à 32 µg/mL. Les espèces cultivées comprenaient 4 souches de Klebsiella pneumoniae et 4 souches d'Escherichia coli, dont 2 souches sensibles et 2 souches résistantes à la colistine pour chacune de ces deux espèces, comprenant 2 souches d'E. coli possédant le gène mcr-1 (24).

Les géloses suivantes ont été préparées en suivant les recommandations du fabricant : Drigalski Lactose Agar (Biokar Diagnostics, Beauvais, France), DifcoTM Violet Red Bile Agar (VRBL) (Beckton-Dickinson, Heidelberg, Allemagne), BBLTM Eosin Methylen Blue Agar (EMB) (Beckton-Dickinson, Heidelberg, Allemagne), BBLTM MacConkey II Agar (Beckton-Dickinson, Heidelberg, Allemagne), le DifcoTM Purple Agar Base additionné de lactose (BCP), et le DifcoTM Purple Agar Base additionné de glucose. Il s'agit de milieux de culture bactérienne sélectifs et chromogéniques utilisés habituellement pour l'isolement des Entérobactéries. Leur différenciation est basée sur la fermentation lactique.

La composition de ces 5 géloses est explicitée ci-après.
a) Milieu Drigalski Lactose Agar (Biokar Diagnostics, Beauvais, France) :
   - agar + bleu de bromothymol et cristal violet ou violet de gentiane + lactose.
b) Milieu VRBL (Violet Red Bile Agar) (Beckton-Dickinson, Heidelberg, Allemagne) :
   - agar+ rouge neutre-cristal violet ou violet de gentiane+ sels biliaires+ lactose.
c) Milieu EMB (Eosin Methylen Blue Agar) (Beckton-Dickinson, Heidelberg, Allemagne) :
   - agar+ Eosine et bleu de méthylène+ lactose.
d) Milieu MacConkey II Agar (Beckton-Dickinson, Heidelberg, Allemagne) :
   - agar+ cristal violet et rouge neutre +lactose le colorant différentie les bactéries lactose + et lactose - et
e) Milieu BCP (Purple Agar Base (Beckton-Dickinson, Heidelberg, Allemagne)) additionné de lactose :
   - agar + pourpre de bromocresol + lactose.
f) Milieu Purple Agar Base (Beckton-Dickinson, Heidelberg, Allemagne)) additionné de glucose :
   - agar +pourpre de bromocresol + glucose.

On entend dans la présente description par « agar », un polymère d'agar-agar à savoir un polymère de galactose (galactane) contenu dans la paroi cellulaire de certaines espèces d'algues rouges appartenant aux familles des Gelidiacées (*Gelidium* et *Pterocladia*) et des Gracilariacées (*Gracilaria*).

1-3-2 Développement du milieu sélectif : choix du sucre fermenté et ajustement de la sélectivité.

Pour permettre la sélectivité de toutes les souches résistantes à la colistine, la concentration en colistine a été ajustée à l'aide de 24 souches représentatives de la plupart des résistances à la colistine (5 souches bactériennes intrinsèquement résistantes, 8 souches d'Entérobactéries comportant des résistantes acquises, 7 souches sensibles servant de contrôle et 4 souches à Gram-positif sensibles à la vancomycine).

Ces différentes souches ont été inoculées sur gélose Purple agar base additionnée de 7.5 g/L de glucose ou de 10 g/L de lactose (laboratoires Humeau, La Chapelle-sur-Erdre, France), et à des concentrations différentes de colistine (0, 4 ou 8 µg/mL) et de vancomycine (0 ou 50 µg/mL).

Pour la sélectivité des souches résistantes à la vancomycine, la concentration de vancomycine a ensuite été adaptée en utilisant 26 souches représentatives de la résistance à la vancomycine (3 genres bactériens intrinsèquement résistants, è souches d'Entérocoques résistantes (VRE), et 15 souches sensibles) (Tableau 2 en annexe), sur des géloses calibrées à 4 µg/mL de colistine et avec des concentrations croissantes de vancomycine (0, 12.5, 25, 50 et 100 µg/mL). Ces différentes concentrations ont également été évaluées avec l'ensemencement de 10 selles.

### 1-3-3 Isolement de souches mcr-1 à partir d'échantillons de selles.

Dix échantillons de selles de poulet en provenance d'Algérie, précédemment criblés en RT-PCR pour le gène mcr-1 (5 selles positives et 5 selles négatives) ont été utilisées (12, 13). Une étape préalable d'enrichissement a été effectuée en inoculant environ 1 g de selle dans un bouillon Trypticase Soja (TSB) (bioMérieux, Marcy-l'Étoile, France), ensuite incubé pendant 24h à 37°C (1). 100 µL de chaque bouillon pur ou dilué ont été déposés puis étalés au râteau sur la gélose Purple agar base additionnée de 7,5 g/L de glucose, 4 µg/mL de colistine et 0, 25, 50, 100 ou 200 µg/mL de vancomycine, incubée à 37°C.

Chaque colonie présentant une morphologie différente a été isolée sur TSA, puis les espèces ont été identifiées par spectrométrie de masse du type MALDI-TOF MS. Pour chaque entérobactérie, la CMI de la colistine a été déterminée par E-test. La présence de mcr-1 a été recherchée par PCR pour chaque isolat résistant à la colistine.

### 1-4 Évaluation du milieu LBJMR selon l'invention.

### 1-4-1 Sensibilité et Spécificité

La sensibilité et la spécificité du milieu LBJMR à détecter toutes les Entérobactéries résistantes à la colistine que ce soit naturellement ou pas acquisition de cette résistance, ont été évaluées sur 76 souches d'Entérobactéries résistantes à la colistine figurant dans la Table 2.

Pour la sélectivité du milieu à détecter les souches à Gram-positifs résistantes à la vancomycine, 11 souches de bactéries à Gram-positifs résistantes à la vancomycine ont été utilisées.

Et, pour l'analyse des souches des espèces bactériennes présentes dans la mucoviscidose, 15 souches ont été utilisées.

Les bactéries ont été ensemencées à 10⁴ UFC sur le milieu LBJMR et sur le milieu TSA en parallèle comme témoin de croissance.

### 1-4-2 Dépistage sur des prélèvements cliniques.

Les ADN de 1063 selles ont été analysés par PCR en temps réel, les positifs ont ensuite été confirmés par PCR standard. Les échantillons dont les ADN ont été positifs pour mcr-1 ont ensuite été ensemencés sur milieu LBJMR. Les souches positives obtenues ont été conservées à -80°C.

1-5 Comparaison entre le milieu LBJMR et d'autres milieux sélectifs contenant des polymyxines.

### 1-5-1 Sensibilité et spécificité.

Pour effectuer ces tests, 30 souches possédant le gène mcr-1, 10 souches d'Entérobactéries sensibles à la colistine et 41 souches résistantes (intrinsèque et acquise) ont été utilisées. Les milieux de culture LBJMR et SuperPolymyxin (19), ont été comparés à d'autres milieux de culture dont le milieu Columbia ANC + 5% de sang de mouton (bioMérieux, Marcy-l'Étoile, France) utilisé pour l'isolement des streptocoques, le milieu Cepacia utilisé pour l'isolement de *Bulkhoderia cepacia* et le milieu EMB (Sigma-Aldricht, Saint-Louis, USA) qui est ici utilisé comme contrôle et qui sert usuellement à l'isolement des coliformes. Les limites de détection ont été déterminées comme décrit précédemment (19).

Enfin, 8 souches d'Entérocoques résistantes à la vancomycine, 3 souches d'espèces bactériennes intrinsèquement résistantes à la vancomycine, 10 souches d'Entérocoques sensibles à la vancomycine, ainsi que les 20 souches bactériennes correspondantes aux prélèvements de patients atteints de mucoviscidose ont été inoculées sur le milieu LBJMR et le milieu SuperPolymyxin de la même manière.

### 1-5-2 Dépistage sur des prélèvements.

Afin de comparer la sélectivité des milieux SuperPolymyxin et LBJMR, 10 selles humaines en provenance de Thaïlande ont été mises en culture. 5 d'entre elles ont donné des cultures positives sur le milieu LBJMR, 5 d'entre elles sont négatives par PCR.

### 1-5-3 Analyse comparative du milieu LBJMR et du milieu SuperPolymyxin.

Afin d'évaluer et de comparer la composition des milieux LBJMR et SuperPolymyxin, 3 géloses de milieu LBJMR ont été modifiées en mélangeant les composants de ces deux milieux : remplacement de la base Purple agar base par l'EMB, remplacement de la vancomycine par la daptomycine (10µg/mL), ajout de l'amphotéricine B (5µg/mL) afin d'étudier leurs effets sur la croissance des Entérobactéries. 30 souches mcr-1 ont été ensemencées sur ces trois milieux en parallèle du milieu LBJMR, du milieu Superpolymyxin et du milieu EMB seul.

Un test complémentaire a ensuite été effectué pour étudier le changement de base d'agar en créant un milieu SuperPolymyxin modifié avec remplacement de l'EMB par le Purple agar base. Ce milieu a été comparé par l'ensemencement de 8 souches mcr-1 (6 souches *E. coli* et 2 souches *K. pneumoniae*), avec le milieu LBJMR, le milieu SuperPolymyxin et le milieu LBJMR modifié par remplacement de son agar.

Enfin, différentes souches ont été inoculées à 104 UFC sur les géloses des milieux LBJMR, SuperPolymyxin et EMB seul ou additionné soit de colistine (4 µg/mL), soit de vancomycine (50 µg/mL), soit des deux. Ces souches étaient composées de 15 souches possédant le gène mcr-1 (5 souches de *K. pneumoniae* et 10 souches d'E *coli*), 5 souches résistantes à la colistine mais par un autre mécanisme (5 souches d'E. coli) et 10 souches sensible à la colistine afin de voir si toutes les souches résistantes à la colistine étaient présentes sur les 2 géloses. En parallèle, ont été testées 7 souches d'Entérocoques résistants à la vancomycine et 10 souches d'Entérocoques sensibles à la vancomycine afin de voir si les géloses permettent une sélectivité des souches résistantes à la vancomycine.

### 1-5-4 Analyses statistiques.

Les données ont été analysées par un test du Chi2 pour une comparaison appariée de la nouvelle gélose sélective et du milieu Cepacia. La signification a été évaluée à p <0,01.

Pour démontrer la corrélation entre les différentes méthodes de détection, un test de Fisher a été réalisée, avec une significativité évaluée à p <0,05.

### 2- Résultats.

### 2-1 Développement du milieu LBJMR selon l'invention.

### 2-1-1 Comparaison des bases d'agar.

Sur les 8 souches testées, l'agar Purple agar base a été retenu pour le milieu LBJMR, remplissant les conditions adéquates avec les plus faibles concentrations de colistine car les souches sensibles étaient inhibées, les bactéries résistantes (CMI>2 µg/mL) ne l'étaient pas (Tableau 3 en annexe). Les autres milieux testés ont été éliminés car avec 8 µg/mL de colistine, le milieu MacConkey a permis la croissance des souches sensibles, et certaines souches résistantes ont été inhibées sur les milieux Drigalski, EMB et VRBL.

Les différentes couleurs des colonies sur la gélose Purple agar base, violet ou jaune, en fonction de la fermentation du glucose étaient intéressantes pour une identification rapide des Entérobactéries présentes dans les échantillons cliniques (Figure 1). Les essais ont ensuite été réalisés sur gélose Purple agar base avec l'ajout de 4 ou 8 µg/mL de colistine et de vancomycine afin d'inhiber les bactéries Gram-positives.

### 2-1-2 Développement du milieu sélectif.

Les tests réalisés sur les 24 souches ont donné les résultats recherchés uniquement pour le Purple agar base ajouté à 4 µg /mL de colistine et 50 µg/ml de vancomycine. En effet, les souches résistantes à la colistine ne sont pas affectées par la présence des deux antibiotiques, alors que les souches sensibles le sont (Tableau A).

**Tableau A : Développement du milieu sélectif pour les souches résistantes à la colistine.**

| **Souches bactériennes** | **CMI de colistine** (µg/mL) | **Agar Purple agar base additionné de colistine + vancomycine (µg/mL)** | | | | | |
|---|---|---|---|---|---|---|---|
| | | **0** | **0+50** | **4+0** | **4+50** | **8+0** | **8+50** |
| **Souches naturellement résistantes à la colistine** | | | | | | | |
| *M. morganii* PM102 | >256 | + | + | + | + | + | + |
| *P. mirabilis* FH112 | >256 | + | + | + | + | + | + |
| *P. vulgaris* | >256 | + | + | + | + | + | + |
| *P. alcalifaciens* TH44 | >256 | + | + | + | + | + | + |
| *S. marcescens* E13 | 128 | + | + | + | + | + | + |

| **Résistance acquise à la colistine** | | | | | | | |
|---|---|---|---|---|---|---|---|
| *E. aerogenes* 1509 | >256 | + | + | + | + | + | + |
| *E. asburiae* LH74 | >256 | + | + | + | + | + | + |
| *E. cloacae* NH52 | >256 | + | + | + | + | + | + |
| *K. pneumonia* LH140 | 96 | + | + | + | + | + | + |
| *K. pneumonia* TH224 | 4 | + | + | + | + | + | + |
| *E. coli* LH257 | 6 | + | + | + | + | + | + |
| *E. coli* P17 | 4 | + | + | + | + | + | + |
| *S. enterica* 100RC3 | 8 | + | + | + | + | + | + |

| **Souches sensibles à la colistine** | | | | | | | |
|---|---|---|---|---|---|---|---|
| *K. 16neumonia* TH20 | 0.125 | + | + | - | - | - | - |
| *P. vulgaris* P100 | 0.94 | + | + | - | - | - | - |
| *S. enterica* | 0.94 | + | + | - | - | - | - |
| *E. asburiae* P113 | 0.19 | + | + | - | - | - | - |
| *E. cloacae* NH151 | 0.50 | + | + | - | - | - | - |
| *E. cloacae* NH74 | 0.38 | + | + | - | - | - | - |
| *E. coli* 169 | 0.096 | + | + | - | - | - | - |

| **Souches à Gram-positif** | | | | | | | |
|---|---|---|---|---|---|---|---|
| *Staphylococcus aureus* | >256 | + | - | + | - | + | - |
| *Enterococcus faecium* | >256 | + | - | + | - | + | - |
| *Enterococcus faecalis* | >256 | + | - | + | - | + | - |
| *Enterococcus gallinarum* | >256 | + | - | + | - | + | - |

| | | | | | | | |
|---|---|---|---|---|---|---|---|
| (+) Croissance (-) Pas de croissance | | | | | | | |

Pour la mise au point de la sélectivité des souches résistantes à la vancomycine, la vancomycine a été fixée à 50 µg/mL après le test sur les 17 souches d'Entérocoques. En effet, à cette concentration la croissance des souches à Gram-positifs résistantes à la vancomycine (CMI>4 µg/mL) est réalisée mais permet l'inhibition des souches sensibles.

Le glucose a été choisi comme substrat glucidique de fermentation en raison de sa meilleure différenciation entre les différentes espèces (les colonies résistantes étaient jaunes contrastées sur fond violet).

La préparation finale du milieu LBJMR selon l'invention est présentée dans le tableau B.

**Tableau B : Composition du milieu LBJMR.**

| Composés | Solutions | Quantité | Concentration finale |
|---|---|---|---|
| Purple Agar Base | | 31 g | 31 g/L (3,10%) |
| Glucose | | 7,5 g | 7,5 g/L (0,75 %) |
| Eau distillée | | 991 mL | |
| Sulfate de Colistine | 1 mg/mL | 4 mL | 4 µg/mL |
| Vancomycine | 10 mg/ml | 5 mL | 50 µg/mL |

### 2-1-3 Isolement de souches mcr-1 à partir d'échantillons de selles.

La mise en culture sur le milieu LBJMR des 10 selles de poulets en provenance d'Algérie a permis l'isolement de souches d'E. coli positives au gène mcr-1 par PCR à partir de 3 des 5 selles positives pour mcr-1. Les seuils de positivité (Ct) des échantillons étaient 26.32, 29.09 et 30.56 avec le système d'amorces PE1, 26,56, 29,8 et 31 avec PE2 (12), et les CMI en colistine de 4, 3 et 4 µg/ml, respectivement pour les souches isolées. Parmi elles, seul l'isolat 235 avait préalablement été détecté sur le milieu Cepacia (13). Les 2 autres selles positives pour mcr-1, avec des valeurs de Ct largement supérieures à 30 cycles obtenues avec les deux systèmes PCR, n'ont pas permis l'isolement d'Entérobactérie, comme pour les dix selles négatives en PCR.

Un test de Fischer a été réalisé pour mettre en évidence la corrélation entre les résultats positifs obtenus par les deux méthodes de détection: RT-PCR et la culture. Le résultat est significatif d'une valeur de 0,02. Il était également important si l'on considère comme positif seulement les deux échantillons positifs avant 30 cycles, avec une valeur p de 0,03.

### 2-2 Évaluation du milieu LBJMR.

### 2-2-1 Sensibilité et Spécificité.

Les 76 Entérobactéries résistantes aux polymyxines ont poussé sur le milieu LBJMR et toutes les bactéries sensibles y ont été inhibées, ce qui lui donne une sensibilité et une spécificité de 100% pour l'isolement des bactéries à Gram-négatifs résistantes à la colistine. L'aspect de ces souches sur le milieu LBJMR est présenté dans la Figure 1. Les 8 Entérocoques résistants à la vancomycine ont pu être détectées sur le milieu LBJMR, contrairement aux Gram-positifs sensibles à la vancomycine ce qui démontre bien la sélectivité du milieu à isoler aussi les bactéries résistantes à la vancomycine.

Des souches provenant de prélèvements de patients atteints par la mucoviscidose, ont poussé ce qui confirme bien la possibilité de pouvoir isoler ce type de bactéries également sur le milieu LBJMR (à l'exception des *Chryséobacterium* qui sont sensibles à la vancomycine).

### 2-2-2 Dépistage sur des prélèvements cliniques.

Parmi les 1063 selles testées, 84 étaient positives en RT-PCR et confirmées en PCR standard. Parmi elles, nous avons mis en culture sur LBJMR les 27 selles qui étaient positives avant 30 cycles en RT-PCR, ainsi que les 24 selles positives entre 30 et 35 cycles et dont les migrations sur gel après PCR standard étaient les plus marquées. Sur 51 échantillons ainsi mis en culture, 13 sont revenus positifs, permettant l'isolement de 14 souches avec le gène mcr-1, dont 12 souches d'*E coli* et 2 souches de *K pneumoniae.* L'un des échantillons, FHM128, a en effet permis l'isolement de deux souches, une souche d'*E coli* et une souche de *K. pneumoniae.* Parmi ces isolats, 4 étaient connus, les souches de K. pneumoniae FHA60 et FHM128, et les souches d'E. *coli* TH99 et P6, isolées lors de précédentes études sur milieu Cepacia (1,18,19). Le milieu LBJMR nous a permis d'isoler 10 nouvelles souches possédant le gène mcr-1, démontrant ainsi son efficacité supérieure au milieu Cepacia dans la détection des souches résistantes à la colistine même à CMI basses. En effet, les CMI de la colistine de ces souches allaient de 2 à 6 µg/mL. (Le fait qu'il y ait si peu de cultures positives en comparaison du nombre de PCR positives s'explique par le fait que les échantillons ont été conservés à -80°c depuis 3 à 4 ans, les bactéries sont possiblement mortes.).

2-3 Comparaison entre le milieu LBJMR selon l'invention et d'autres milieux sélectifs contenant des polymyxines.

### 2-3-1 Sensibilité et Spécificité par rapport aux autres milieux.

Les résultats de tous les milieux testés sont résumés dans le Tableau C. Sur les 33 souches possédant le gène mcr-1, toutes les souches ont poussé sur le milieu LBJMR, le milieu SuperPolymyxin, et le milieu EMB avec une limite de détection de 10¹ UFC/mL. Les souches sensibles ont été inhibées sur chaque gélose. En revanche, les germes intrinsèquement résistants M. morganii et P. alcalifaciens utilisés comme contrôle ont été inhibés sur SuperPolymyxin. À 10⁴ UFC de bactéries, seulement la moitié des souches possédant le gène mcr-1 sont cultivées sur le milieu Cepacia (tableau D). En considérant les 21 souches possédant le gène mcr-1 mais présentant une CMI faible (<8 µg/mL), seulement 5 d'entre elles ont poussé sur le milieu Cepacia. Les sensibilités des milieux LBJMR (100%) et Cepacia (47%), ont été comparées à l'aide d'un test statistique Chi2, le milieu LBJMR est significativement plus sensible que Cepacia. Le milieu EMB a servi de témoin de croissance, celui-ci n'étant pas sélectif, et toutes les souches ont pu croître dessus, mêmes aux plus basses concentrations. La gélose CNA n'était pas adaptée, avec la croissance de seulement deux souches possédant le gène mcr-1 en raison de la présence de l'acide nalidixique.

**Tableau C : Comparaison de la sensibilité de différents milieux de culture contenant de la colistine pour l'isolement d'Entérobactéries résistantes à la colistine.**

| **Souches bactériennes** | **Nombre de souches** | **Milieux de culture** | | | | | | | |
|---|---|---|---|---|---|---|---|---|---|
| | | **LBJMR** | **SP^{a}** | **Cepacia** | **EMB^{b}** | **ANC^{C}** | **(1)** | **(2)** | **(3)** |
| ***Souches mcr-1 positives*** | | | | | | | | | |
| *E. coli* | 23 | 23 | 23 | 14 | 23 | 4 | 23 | 23 | 0 |
| *K. pneumoniae* | 7 | 7 | 7 | 7 | 7 | 0 | 7 | 7 | 7 |

| ***Souches résistantes intrinsèquement à la colistine*** | | | | | | | | | |
|---|---|---|---|---|---|---|---|---|---|
| *M. morganii* | 1 | 1 | 0 | 1 | 1 | 0 | 1 | 1 | 1 |
| *S. marcescens* | 1 | 1 | 1 | 1 | 1 | 0 | 1 | 1 | 1 |
| *P. mirabilis* | 1 | 1 | 1 | 1 | 1 | 0 | 1 | 1 | 1 |
| *P. vulgaris* | 1 | 1 | 1 | 1 | 1 | 0 | 1 | 1 | 1 |
| *P. alcalifaciens* | 1 | 1 | 0 | 0 | 1 | 0 | 1 | 1 | 1 |

| ***Souches sensibles à la colistine*** | | | | | | | | | |
|---|---|---|---|---|---|---|---|---|---|
| *E. coli* | 2 | 0 | 0 | 0 | 0 | 0 | 0 | 0 | 0 |
| *K. pneumoniae* | 3 | 0 | 0 | 0 | 0 | 0 | 0 | 0 | 0 |
| *S. enterica* | 2 | 0 | 0 | 0 | 0 | 0 | 0 | 0 | 0 |
| *E. cloacae* | 1 | 0 | 0 | 0 | 0 | 0 | 0 | 0 | 0 |
| *E. asburiae* | 1 | 0 | 0 | 0 | 0 | 0 | 0 | 0 | 0 |
| *P. vulgaris* | 1 | 0 | 0 | 0 | 0 | 0 | 0 | 0 | 0 |

| | | | | | | | | | |
|---|---|---|---|---|---|---|---|---|---|
| (SP^{a}) : Milieu SuperPolymyxin ; (EMB^{b}) : Eosine - Methylene Blue; (ANC^{c}) : Acide Nalidixique - Colistine ; (1) : Purple Agar Base + glucose + colistine + daptomycine ; (2) : Purple Agar Base + glucose + colistine + vancomycine + amphotéricine B ; (3) : EMB Agar + colistine + vancomycine. | | | | | | | | | |

**Tableau D : Comparaison de la sensibilité des milieux de culture LBJMR et Cepacia. L'inoculum de chaque souche a été calibré à 10⁴ UFC.**

| **Souches testées** | | | **Nombre de souches ayant poussé** | | |
|---|---|---|---|---|---|
| Mécanisme de résistance à la colistine | Nombre de souches | CMI de colistine (µg/mL) | LBJMR | Cepacia | TSA (témoin) |
| ***mcr-1*** | 21 | <8 | 21 (100%) | 5 (23,8%) | 21 (100%) |
| | 9 | ≥8 | 9 (100%) | 9(100%) | 9(100%) |
| Total | 30 | | 30(100%) | 14 (46,7%) | 30(100%) |
| Autres* | 41 | | 41(100%) | 41(100%) | 41(100%) |

| | | | | | |
|---|---|---|---|---|---|
| * souches résistantes à la colistine mais par un autre mécanisme différent de mcr-1. | | | | | |

### 2-3-2 Analyse comparative du milieu LBJMR et du milieu SuperPolymyxin.

Concernant les Entérocoques résistants à la vancomycine, ils ont été inhibés sur le milieu SuperPolymyxin (car présence de daptomycine) mais pas sur le milieu LBJMR (tableau E). Le milieu LBJMR possède donc comme avantage par rapport au milieu SuperPolymyxin de pouvoir également isoler spécifiquement les souches résistantes à la vancomycine.

Enfin, les résultats de culture de selles sur les 2 milieux avaient la même sensibilité pour les souches Gram-négatives résistantes à la colistine mais seul le milieu LBJMR permettait la détection des Gram-positifs résistants à la vancomycine. (Tableau E). Exemple Figure 1 : à partir d'un prélèvement biologique positif à *Entérococcus faecium* résistant à la vancomycine, nous avons pu isoler 2 souches d'Entérobactéries sur le milieu LBJMR uniquement et surtout nous avons pu aussi détecter la présence 2 souches dont une de K. pneumoniae et une souche d'E *coli* résistantes à la colistine. Le milieu LBJMR a donc permis à partir d'un même prélèvement clinique de détecter à la fois 2 souches à Gram-négatif résistantes à la colistine et une souche à Gram-positif résistante à la vancomycine.

Les avantages du milieu LBJMR en comparaison avec le milieu SuperPolymyxin sont :
- Sensibilité de 100% pour l'isolement des Entérocoques résistants à la vancomycine ;
- Visualisation facile et rapide de la croissance des bactéries d'intérêt ;
- Isolement des bactéries présentes chez les patients atteints de mucoviscidose ;
- Visualisation facilitée des souches d'*E. coli* ;
- Spécificité de 100% pour les germes résistants à la colistine ; et
- Possibilité d'isoler sur la même gélose des Entérocoques résistants à la vancomycine et des Bacilles à Gram-négatif résistants à la colistine (Figure 1).

**Tableau E : Comparaison des géloses et limite de détection des Gram-positifs (UFC/mL)**

| **Souches bactériennes** | **Isolats** | **CMI (µg/mL)** : | LBJMR | Superpolymyxin |
|---|---|---|---|---|
| **Gram-positifs résistants à la vancomycine** | | **vancomycine** | | |
| *E. faecium* | DSM 17050 | >256 | 10¹ | >10⁵ |
| *E. faecium* | DSM 13590 | >256 | 10¹ | >10⁵ |
| *E. faecium* | DSM 25698 | >256 | 10² | >10⁵ |
| *E. faecium* | DSM 25697 | >256 | 10¹ | >10⁵ |
| *E. faecium* | VRE | 24 | 10⁴ | >10⁵ |
| *E. faecium* | FHM-VRE1 | >256 | 10³ | >10⁵ |
| *E. faecium* | FHM-VRE2 | >256 | 10¹ | >10⁵ |
| *E. faecalis* | JH2-2 : Tn 1549 | 64 | 10³ | >10⁵ |
| *W. cibaria* | P18A | >256 | 10¹ | >10⁵ |
| *W. confusa* | P18B | >256 | 10¹ | >10⁵ |
| *L. lactis* | P18C | >256 | 10¹ | >10⁵ |

| **Gram-positifs sensibles à la vancomycine** | | **vancomycine** | | |
|---|---|---|---|---|
| *S. aureus* | CF_Marseille | 1 | >10⁵ | >10⁵ |
| *S. aureus* | FHM-SA | NC | >10⁵ | >10⁵ |
| *E. faecium* | TH26 | 0.75 | >10⁵ | >10⁵ |
| | 349 | 0.75 | >10⁵ | >10⁵ |
| *E. faecium* | TH12 | 2 | >10⁵ | >10⁵ |
| *E. faecium* | LH165 | 0.75 | >10⁵ | >10⁵ |
| *E. faecium* | A17 | 0.75 | >10⁵ | >10⁵ |
| *E. faecium* | TH43 | 0.75 | >10⁵ | >10⁵ |
| *E. faecium* | TH95 | 0.75 | >10⁵ | >10⁵ |
| *E. faecium* | 282 | 0.75 | >10⁵ | >10⁵ |
| *E. faecalis* | JH2-2 : C2 | 3 | >10⁵ | >10⁵ |
| *E. faecalis* | JH2-2S | 2 | >10⁵ | >10⁵ |
| *E. gallinarum* | SE3 | 1.5 | >10⁵ | >10⁵ |
| *E. casseliflavus* | SE3 | 1 | >10⁵ | >10⁵ |
| *E. hirae* | LH111 | 1.5 | >10⁵ | >10⁵ |

| **Gram-négatifs non-fermentants liés à la mucoviscidose résistantes à la colistine** | | | | |
|---|---|---|---|---|
| **Colistine** | | | | |
| *B. cepacia* | FHM-BC1 | >256 | 10¹ | 10¹ |
| *B. cepacia* | FHM-BC2 | 24 | 10¹ | 10⁵ |
| *A. xylosoxidans* | FHM-AX | 3 | 10¹ | >10⁵ |
| *O. anthropi* | FHM-OA | NC | 10² | >10⁵ |
| *I. limosus* | FHM-IL | >256 | 10⁴ | 10⁴ |
| *S. maltophilia* | FHM-SM | 12 | 10³ | >10⁵ |
| *P. pulmonicola* | FHM-PP | >256 | 10¹ | 10³ |
| *P. aeruginosa* | FHM-PACOLR1 | >256 | 10¹ | 10¹ |
| *P. aeruginosa* | FHM-PACOLR2 | NC | 10⁵ | >10⁵ |
| *P. aeruginosa* | FHM-PACOLR3 | NC | 10² | >10⁵ |
| *S. putrefaciens* | FHM-SP | NC | 10³ | >10⁵ |
| *A. baumannii* | AB-COLR1 | 8 | 10² | 10¹ |

| **Gram-négatifs non-fermentants liés à la mucoviscidose sensibles à la colistine** | | | | |
|---|---|---|---|---|
| *P. aeruginosa* | FHM-PA4 | 2 | >10⁵ | >10⁵ |
| *P. aeruginosa* | FHM-PA5 | 0.50 | >10⁵ | >10⁵ |
| *P. aeruginosa* | FHM-PA6 | 0.38 | >10⁵ | >10⁵ |
| *S. xianemensis* | 111P | 0.125 | >10⁵ | >10⁵ |
| *S. xianemensis* | 111A | 0.094 | >10⁵ | >10⁵ |

### 2-3-3 Mise en évidence de la synergie.

Les cultures sur les différents milieux composés ont mis en évidence une inhibition systématique des souches d'E. coli positives pour le gène mcr-1 sur la gélose EMB ajoutées à de la colistine et de la vancomycine, alors que les K. pneumoniae étaient bien présentes. Pour les autres milieux LBJMR modifiés, la sensibilité était de 100% pour les souches résistantes à la colistine. Ainsi, une potentielle synergie a été mise en évidence entre les composants de la gélose EMB (éosine et/ou bleu de méthylène) avec les antibiotiques testés (vancomycine et/ou colistine), empêchant la croissance des souches *E. coli* possédant le gène mcr-1 (Tableaux C et F).

En explorant cette piste, ce résultat a été imputé à l'effet de la colistine ou de la vancomycine seule. En effet la sensibilité de la gélose EMB additionnée de colistine était de 100% également, tandis que celle qui ne contient que la vancomycine seule a permis la croissance de toutes les Entérobactéries. En revanche, l'addition des deux antibiotiques à la gélose EMB a de nouveau montré l'inhibition des souches d'E *coli* mcr-1 positive, mais pas de souches d'*E coli* résistantes à la colistine mais possédant un mécanisme différent (Tableau F). La synergie est donc efficace sur les souches ayant une CMI basse de la colistine, et concerne l'addition de colistine et de vancomycine ensemble avec la gélose EMB. Si l'on ne peut imputer cette inhibition à la seule présence de la vancomycine, on remarque que les souches d'*E coli* ne donnent plus leur aspect métallique (vert scarabée), mais sont roses comme les souches *K. pneumoniae.* Il existe donc une altération due à la vancomycine.

Concernant les Gram-positifs, les résultats sont confirmés : le milieu LBJMR est sensible à 100% pour l'isolement des Entérocoques résistants à la vancomycine, alors que le milieu SuperPolymyxin les inhibe tous (sensibilité des souches à la daptomycine). On relève une sensibilité diminuée de la gélose EMB additionnée de vancomycine seule ou associée avec la colistine. En effet seuls les Entérocoques résistants à la vancomycine avec une CMI>256 µg/mL ont pu être détectées. Enfin, l'ajout seul de colistine n'a pas affecté leur croissance. La composition du milieu LBJMR apparait donc supérieure ici.

**Tableau F : Étude de synergie entre la colistine, la vancomycine et la gélose EMB.**

| | | | | | | | |
|---|---|---|---|---|---|---|---|
| La colistine est la vancomycine ont été ajoutés, seuls ou ensemble, aux mêmes concentrations que dans le LBJMR, 4 et 50 µg/mL, respectivement. Les nombres indiquent le nombre de souches ayant poussé sur les géloses dans chaque catégorie. | | | | | | | |

| **Souches** | **Nombre** | **LBJMR** | **SP** | **EMB VCN** | **EMB CT** | **EMB VCN CT** | **EMB** |
|---|---|---|---|---|---|---|---|
| *K. pneumoniae mcr-1* | 5 | 5 | 5 | 5 | 5 | 5 | 5 |
| *E. coli mcr-1* | 10 | 10 | 10 | 9 | 9 | 2 | 10 |
| *E. coli R non mcr-1* | 5 | 5 | 5 | 4 | 4 | 4 | 5 |
| *E. coli sensibles* | 10 | 0 | 0 | 8 | 0 | 0 | 10 |
| *E. faecium ERV* | 6 | 6 | 0 | 4 | 6 | 4 | 6 |
| *E. faecalis ERV* | 1 | 1 | 0 | 0 | 1 | 0 | 1 |
| *E. faecium sensible* | 8 | 0 | 0 | 0 | 8 | 0 | 8 |
| *E. faecalis sensible* | 2 | 0 | 0 | 0 | 2 | 0 | 2 |
| *W. cibaria* | 1 | 1 | 0 | 1 | 1 | 1 | 1 |

| | | | | | | | |
|---|---|---|---|---|---|---|---|
| SP : SuperPolymyxin, EMB : Eosine - Methylene Blue, VCN: Vancomycine, CT: Colistine | | | | | | | |

### ANNEXE : TABLEAUX 1 à 3

**Tableau 1. Comparaison des différents milieux de culture contenant des polymyxines. Composition en antibiotiques : Polymyxine B (B), Colistine (C), Amphotéricine B (AB), Anisomycine (A) Cycloheximide (CH), Nystatine (N)**

| **Milieux** | **Composition en aqents sélectifs (µg/mL)** | | | | **Utilisation** | **Référence** |
|---|---|---|---|---|---|---|
| | **Gram-négatifs** | | **Gram-positifs** | **Levures** | | |
| | **Polymyx ine** | **Autres** | | | | |
| LBJMR^{a} | 4 (C) | | Vancomycine 50 | | Gram-négatifs colistine-résistants Gram-positifs vancomycine-résistants | Cette étude |
| SuperPolymyxin | 3,5 (C) | | Daptomycine 10 | 5 (AB) | Entérobactérie s colistine-résistantes | Nordmann *et al.,* 2016 |
| | | | Bleu de méthylène 65 | | | |
| | | | Éosine 400 | | | |
| Martin-Lewis Agar | 7,5 (C) | Triméthoprime 5 | Vancomycine 4 | 20 (A) | *Neisseria sp.* | Martin and Lewis, 1977 |
| Thayer-Martin Agar | 7,5 (C) | | Vancomycine 3 | 2,57 (N) | *Neisseria sp.* | Thayer and Martin, 1966 |
| MTM^{b} Agar | 7,5 (C) | Triméthoprime 5 | Vancomycine 3 | 2,57 (N) | *Neisseria sp.* | Martin and Lester, 1971 |
| NYC^{c} Agar | 7,5 (C) | Triméthoprime 3 | Vancomycine 2 | 20 (A) | *Neisseria sp.* | Fauer *et al.,* 1973 |
| Cepacia | 35,7 (B) | | Ticarcilline 100 | | *Bulkhoderia cepacia* | Gilligan *et al.,* 1985 |
| OFPBL^{d} Agar | 35,7 (B) | | Bacitracine 2,7 | | *Bulkhoderia cepacia* | *Welch, 1987* |
| Bulkhoderia Cepacia Agar | 17.8 (B) | Gentamicine 5 | Ticarcilline 100 | | *Bulkhoderia cepacia* | Gillian *et al.,* 1985 |
| Bulkhoderia Cepacia Agar Selectif | 71.4 (B) | Gentamicine 10 | Vancomycine 2.5 | | *Bulkhoderia cepacia* | Henry, 1997 |
| CNA^{e} | 10 (C) | Acide nalidixique 10 | | | *Streptococcus sp.* et autres Gram-positifs | Ellner *et al.,* 1966 |
| BCYE^{f} Selective Agar with GPVC^{g} | 9,4 (B) | Glycine 3000 | Vancomycine 1 | 80 (CH) | *Legionella sp.* | Dennis *et al.,* 1984 |
| BCYE Selective Agar with CCVC^{h} | 16 (C) | | Vancomycine 0,5 Cefalotine 4 | 80 (CH) | *Legionella sp.* | Bopp et al., 1981 |
| BCYE Agar Selectif avec GPVAⁱ | 11,9 (B) | Glycine 3000 | Vancomycine 1 | 80 (A) | *Legionella sp.* | Ta et al., 1995 |
| BCYE Agar Selectif avec PAV^{j} | 4.76 (B) | | Vancomycine 0,5 | 80 (A) | *Legionella sp.* | Stout *et al.,* 1982 |
| BCYE Agar Selectif avec PAC^{k} | 9.52 (B) | | Cefamandole 2 | 80 (A) | *Legionella sp.* | Edelstein, 2007 |
| BCYE Agar Selectif avec DGVP^{l} | 9.4 (B) | Glycine 3000 | Vancomycine 1 | | *Legionella sp.* | Murray, 2007 |
| CPC^{m} | 66.34 (C) 11.9 (B) | | | | *Vibrio sp.* | Vanderzant and Splittstoesser, 1992 |
| Campylobacter Agar Butzler | 0.3 (C) | Novobiocine 5 | Cephazoline 15 Bacitracin 337.8 | 50 (CH) | *Campylobacter sp.* | Butzler, 1973 |
| Campylobacter Agar Skirrow | 0.3 (B) | Triméthoprime 5 | Vancomycine 10 | | *Campylobacter sp.* | Skirrow, 1977 |
| Campylobacter Agar Blaser-Wang | 0.3 (B) | Triméthoprime 5 | Vancomycine 10 Cefalotine 15 | 2 (AB) | *Campylobacter sp.* | Blaser *et al.,* 1978 |
| Campylobacter Agar Preston | 0.3 (B) | Triméthoprime 5 | Rifampicine 5 | 50 (CH) | *Campylobacter sp.* | Bolton and Robertson, 1982 |
| Brucella selectif | 1 (B) | | Bacitracine 500 | 100 (CH) | *Brucella spp.* | Jones and Brinley, 1958 |
| Oxford | 20 (C) | Fosfomycine 10 | Cefotetan 2 Acriflavine 5 | 400 (CH) | *Listeria monocytogene s* | Curtis *et al.,* 1989 |
| Oxford modifié | 10 (C) | | Moxalactam 20 | | *Listeria monocytogene* s | Lee *et al.,* 1989 |
| PALCAMⁿ | 10 (B) | | Ceftazidime 8 Acriflavine 5 | | *Listeria spp.* | van Nerren *et al.,* 1989 |
| MYP^{o} | 11.9 (B) | | | | *Bacillus cereus* | Mossel, 1967 |
| Middlebrook 7H11 Agar | 2.38 (B) | Triméthoprime 20 | Carbenicilline 50 | 20 (AB) | *Mycobacteriac eae* | Cohn *et al,* 1968 |
| SPS^{p} Agar | 10 (B) | Sulfadiazine 120 | | | *Clostridium perfringens* | Angelotti *et al,* 1962 |
| TSN^{q} Agar | 20 (B) | Néomycine 50 | | | *Clostridium perfringens* | Marshall *et al.,* 1965 |
| SFP^{r} Agar | 3,57 (B) | Kanamycine 12 | | | *Clostridium perfringens* | Shahidi and Ferguson, 1971 |
| LB modifié | 2 (C) | | Vancomycine 50 | | Gram-négatifs colistine-résistants | Von Wintersdorff *et al.,* 2016 |
| MacConkey modifié | 2 (C) | | | | Gram-négatifs colistine-résistants | Meinersmann *et al.,* 2016 |

| | | | | | | |
|---|---|---|---|---|---|---|
| a LBJMR : Notre milieu de culture, bMTM : Modified Thayer-Martin, NYCc : New York City, dOFPBL : Oxidation/Fermentation, Polymyxine B, Bacitracine, Lactose, eCNA : Colistine, Acide nalidixique, fBCYE : Buffered Charcoal, Yeast Extract, gGPVC : Glycine, Polymyxine B, Vancomycine et Cycloheximide, hCCVC : Cephalotine, Colistine, Vancomycine et Cycloheximide, iGPVA : Glycine, Polymyxine B, Vancomycine et Anisomycin, jPAV : Polymyxine B, Anisomycine, Vancomycine, kPAC : Polymyxine B, Anisomycine, Céfamandole, IDGVP : Dyes, Glycine, Vancomycine, Polymyxine B, mCPC : Cellobiose, Polymyxine B, Colistine, nPALCAM : Polymyxine B, Acriflavine, Lithium, Ceftazidime, Esculine, Mannitol, oMYP : Mannitol, Egg Yolk, Polymyxine B, pSPS : Sulfite, Polymyxine B, Sulfadizine, qTSN : Trypticase , Sulfite, Néomycine, rSFP : Shahidi Ferguson Perfringens. | | | | | | |

**Tableau 2. Liste des souches étudiées et leurs Concentrations Minimales Inhibitrices (CMI) pour la colistine (Gram-négatifs) ou la vancomycine (Gram-positifs).**

| **Souches bactériennes** | **Isolats** | **Origine** | **Echantillons** | **CMI** | **Mécanismes de résistance** | **Références** |
|---|---|---|---|---|---|---|
| *Entérobactéries* **avec une résistance acquise à la colistine** | | | | | | |
| *Escherichia coli* | SE65 | Algérie | Humain | 4 | *mcr-1* | (Berrazeg et al., 2016) |
| | 117R | Arabie saoudite | Humain | 4 | *mcr-1* | (Leangapichart et al., 2016) |
| | 1R | Arabie saoudite | Humain | 4 | *mcr-1* | (Leangapichart et al., 2016) |
| | 1R 2104 | Arabie saoudite | Humain | 4 | *mcr-1* | (Leangapichart et al., 2016) |
| | 44A | Arabie saoudite | Humain | 4 | *mcr-1* | (Leangapichart et al., 2016) |
| | 6R | Arabie saoudite | Humain | 4 | *mcr-1* | (Leangapichart et al., 2016) |
| | 85R | Arabie saoudite | Humain | 4 | *mcr-1* | (Leangapichart et al., 2016) |
| | 95R | Arabie saoudite | Humain | 4 | *mcr-1* | (Leangapichart et al., 2016) |
| | 96R | Arabie saoudite | Humain | 4 | *mcr-1* | (Leangapichart et al., 2016) |
| | 134R | Arabie saoudite | Humain | 3 | *mcr-1* | (Leangapichart et al., 2016) |
| | 143R | Arabie saoudite | Humain | 3 | *mcr-1* | (Leangapichart et al., 2016) |
| | LH121 | Laos | Humain | 16 | *mcr-1* | (Olaitan et al., 2016) |
| | LH140 | Laos | Humain | 12 | *mcr-1 PhoP* E375K | (Olaitan et al., 2016) |
| | LH257 | Laos | Humain | 12 | *mcr-1* | (Olaitan et al., 2016) |
| | LH57 | Laos | Humain | 8 | *mcr-1 PhoP* E375K | (Olaitan et al., 2016) |
| | LH1 | Laos | Humain | 6 | *mcr-1* | (Olaitan et al., 2016) |
| | LH30 | Laos | Humain | 6 | *mcr-1* | (Olaitan et al., 2016) |
| | TH214 | Thaïlande | Humain | 6 | *mcr-1* | (Olaitan et al., 2016) |
| | TH99 | Thaïlande | Humain | 4 | *mcr-1* | (Olaitan et al., 2016) |
| | FHA102 | France | Humain | 12 | *pmrA* A159V | (Olaitan et al., 2016) |
| | FHM19 | France | Humain | 12 | *pmrA* P7-Q12 (del 6aa) | (Olaitan et al., 2016) |
| | FHA113 | France | Humain | 12 | *pmrA* T156K | (Olaitan et al., 2016) |
| | NH94 | Nigeria | Humain | 12 | *pmrA* I92 insertion | (Olaitan et al., 2016) |
| | TH176 | Thaïlande | Humain | 6 | Inconnu | (Olaitan et al., 2016) |
| | LB4 | France | Humain | 16 | Inconnu | De cette étude |
| | 235 | Algérie | Poulet | 4 | *mcr-1* | (Olaitan et al., 2016) |
| | SA9 | Algérie | Poulet | 3 | *mcr-1* | De cette étude |
| | SE3 | Algérie | Poulet | 3 | *mcr-1* | De cette étude |
| | P6 | Laos | Cochon | 6 | *mcr-1* | (Olaitan et al., 2016) |
| | P10 | Laos | Cochon | 4 | *mcr-1* | (Olaitan et al., 2016) |
| | P17 | Laos | Cochon | 4 | *mcr-1* | (Olaitan et al., 2016) |
| *Klebsiella pneumoniae* | FHA60 | France | Humain | 8 | *mcr-1* | (Olaitan et al., 2014; Rolain et al., 2016) |
| | FHM128 | France | Humain | 4 | *mcr-1* | (Olaitan et al., 2014; Rolain et al., 2016) |
| | 119R | Arabie saoudite | Humain | 3 | *mcr-1* | (Leangapichart et al., 2016) |
| | LH131 | Laos | Humain | 32 | *mcr-1 mgrB* Stop | (Olaitan et al., 2014; Rolain et al., 2016) |
| | LH61 | Laos | Humain | 24 | *mcr-1 mgrB* Sub | (Olaitan et al., 2014; Rolain et al., 2016) |
| | LH17 | Laos | Humain | 12 | *mcr-1 pmrB* T157P | (Olaitan et al., 2014; Rolain et al., 2016) |
| | LH92 | Laos | Humain | 12 | *mcr-1* | (Olaitan et al., 2014; Rolain et al., 2016) |
| | LB1 | France | Humain | 32 | *mgrB* Stop | Non publié |
| | FHM169b | France | Humain | 8 | *mgrB* Stop | (Olaitan et al., 2014) |
| | LH12 | Laos | Humain | 32 | *mgrB* Stop *pmrA* E35A | (Olaitan et al., 2014) |
| | LH74 | Laos | Humain | 12 | *mgrB* sub | (Olaitan et al., 2014) |
| | NH53 | Nigeria | Humain | 4 | *mgrB* Sub | (Olaitan et al., 2014) |
| | TH20 | Thaïlande | Humain | 32 | *mgrB* IS 1 | (Olaitan et al., 2014) |
| | TH196 | Thaïlande | Humain | 8 | *mgrB* IS 1 | (Olaitan et al., 2014) |
| | TH28 | Thaïlande | Humain | 8 | *mgrB* IS2 | (Olaitan et al., 2014) |
| | TH213 | Thaïlande | Humain | 12 | *mgrB* IS3 | (Olaitan et al., 2014) |
| | LH70 | Laos | Humain | 12 | pmrA G53C | (Olaitan et al., 2014) |
| | TH34 | Thaïlande | Humain | 24 | *pmrB* S205P | (Olaitan et al., 2014) |
| | TH54 | Thaïlande | Humain | 16 | *pmrB* T157P | (Olaitan et al., 2014) |
| | TH224 | Thaïlande | Humain | 4 | *pmrB* T157P | (Olaitan et al., 2014) |
| | FHM120b | France | Humain | 64 | Inconnu | (Olaitan et al., 2014) |
| | FHA105 | France | Humain | 24 | Inconnu | (Olaitan et al., 2014) |
| | FHM77 | France | Humain | 8 | Inconnu | (Olaitan et al., 2014) |
| | LH140 | Laos | Humain | 16 | Inconnu | (Olaitan et al., 2014) |
| | LH94 | Laos | Humain | 16 | Inconnu | (Olaitan et al., 2014) |
| | LH375 | Laos | Humain | 12 | Inconnu | (Olaitan et al., 2014) |
| | LH102 | Laos | Humain | 8 | Inconnu | (Olaitan et al., 2014) |
| | TH205 | Thaïlande | Humain | 64 | Inconnu | (Olaitan et al., 2014) |
| | TH68 | Thaïlande | Humain | 64 | Inconnu | (Olaitan et al., 2014) |
| | TH176 | Thaïlande | Humain | 12 | Inconnu | (Olaitan et al., 2014) |
| | TH21 | Thaïlande | Humain | 12 | Inconnu | (Olaitan et al., 2014) |
| | TH114 | Thaïlande | Humain | 8 | Inconnu | (Olaitan et al., 2014) |
| | TH164 | Thaïlande | Humain | 6 | Inconnu | (Olaitan et al., 2014) |
| | TH166 | Thaïlande | Humain | 3 | Inconnu | (Olaitan et al., 2014) |
| | LB3 | France | Humain | 64 | Inconnu | De cette étude |
| *Klebsiella oxytoca* | FHA41 | France | Humain | 12 | *mgrB* IS 1 | (Olaitan et al., 2014; Olaitan and Rolain, 2015) |
| | FHA124 | France | Humain | 6 | Inconnu | (Olaitan et al., 2014) |
| *Enterobacter aerogenes* | EA15090 | France | Humain | >256 | *PmrA* | (Diene et al., 2013) |
| *Enterobacter asburiae* | LH74 | Laos | Humain | > 256# | Inconnu | Non publié |
| *Enterobacter cloacae* | NH131 | Nigeria | Humain | 64# | Inconnu | Non publié |
| | NH132 | Nigeria | Humain | > 256# | Inconnu | Non publié |
| | NH52 | Nigeria | Humain | > 256# | Inconnu | Non publié |
| | SB1 | France | Humain | 2 | *mcr-1* | Non publié |
| *Salmonella enterica* | 65RC | Arabie saoudite | Humain | 16 | Inconnu | (Olaitan et al., 2015) |
| | 100RC3 | Arabie saoudite | Humain | 8 | Inconnu | (Olaitan et al., 2015) |

| ***Entérobactéries* avec une résistance intrinsèque à la colistine** | | | | | | |
|---|---|---|---|---|---|---|
| *Proteus mirabilis* | FH112 | France | Humain | >256 | Intrinsèque | |
| *Proteus vulgaris* | PV148 | France | Humain | >256 | Intrinsèque | |
| *Providencia alcalifaciens* | TH44 | Thaïlande | Humain | >256 | Intrinsèque | |
| *Providencia rettgeri* | H1734 | France | Humain | >256 | Intrinsèque | |
| *Morganella morganii* | FM102 | France | Humain | >256 | Intrinsèque | |
| *Serratia marcescens* | E13 | Laos | Environne ment | 128 | Intrinsèque | |

| **Gram-négatifs non fermentants liés à la mucoviscidose** | | | | | | |
|---|---|---|---|---|---|---|
| *Burkholderia cepacia* | FHM-BC1 | France | Humain | 24 | Inconnu | |
| | FHM-BC2 | France | Humain | >256 | Inconnu | |
| *Chryseobacterium gleum* | FHM-CG | France | Humain | >256 | Intrinsèque | |
| *Chryseobacterium oranimense* | FHM-CO | France | Humain | >256 | Intrinsèque | |
| *Chryseobacterium indologenes* | FHM-CI | France | Humain | >256 | Intrinsèque | |
| *Brevundimonas diminuta* | FHM-BD | France | Humain | 32 | Inconnu | |
| *Achromobacter xyloso xidans* | FHM-AX | France | Humain | 3 | Inconnu | |
| *Stenotrophomonas maltophilia* | FHM-SM | France | Humain | 12 | Inconnu | |
| *Inquilinus limosus* | FHM-IL | France | Humain | >256 | Intrinsèque | |
| *Pandoraea pulmonicola* | FHM-PP | France | Humain | >256 | Inconnu | |
| *Shewanella putrefaciens* | FHM-SP | France | Humain | NC | Intrinsèque | |
| *Ochromobacter anthropi* | FHM-OA | France | Humain | NC | Intrinsèque | |
| *Pseudomonas aeruginosa* | FHM-PACOLR1 | France | Humain | >256 | Inconnu | |
| | FHM-PACOLR2 | France | Humain | NC | Inconnu | |
| | FHM-PACOLR3 | France | Humain | NC | Inconnu | |

| **Autres gram-négatifs non-fermentants résistant à la colistine** | | | | | | |
|---|---|---|---|---|---|---|
| *Acinetobacter baumannii* | AB-COLR1 | France | Humain | 8 | Inconnu | |
| | AB-COLR2 | France | Humain | NC | Inconnu | |
| *Acinetobacter nosocomialis* | ABG13R | France | Humain | NC | Inconnu | |

| **Gram-négatifs sensibles à la colistine** | | | | | | |
|---|---|---|---|---|---|---|
| *Enterobacter asburiae* | P113 | France | Humain | 0,19 | Sensible | |
| *Enterobacter cloacae* | NH151 | Nigeria | Humain | 0,50 | Sensible | |
| | NH74 | Nigeria | Humain | 0,38 | Sensible | |
| *Klebsiella pneumoniae* | CIP 82.91 | | Humain | 0,125 | Sensible | |
| | LB2 | France | Humain | 0,128 | Sensible | |
| | TH20S | Thaïlande | Humain | 0,125 | Sensible | |
| | TH28S | Thaïlande | Humain | 0,125 | Sensible | |
| *Proteus vulgaris* | P100 | Algérie | Humain | 0,94 | Sensible | |
| *Salmonella enterica* | 108R | Arabie saoudite | Humain | 1 | Sensible | (Olaitan et al., 2015) |
| | 122R | Arabie saoudite | Humain | 0,5 | Sensible | (Olaitan et al., 2015) |
| *Escherichia coli* | ATCC25922 CIP 76.24 | | | 0,094 | Sensible | |
| | 161 | Algérie | Poulet | 0,094 | Sensible | |
| | 169 | Algérie | Poulet | 0,094 | Sensible | |
| | FHM88S | France | Humain | 0,125 | Sensible | De cette étude |
| | TH134S | France | Humain | 0,094 | Sensible | De cette étude |
| | LH53S | Laos | Humain | 0,094 | Sensible | De cette étude |
| | LH165S | Laos | Humain | 0,074 | Sensible | De cette étude |
| | TH77S | Thaïlande | Thaïlande | 0,064 | Sensible | De cette étude |
| | 282S | Algérie | Poulet | 0,074 | Sensible | De cette étude |
| | FHM19S | France | Humain | 0,125 | Sensible | De cette étude |
| *Pseudomonas aeruginosa* | FHM-PA4 | France | Humain | 2 | Sensible | |
| | FHM-PA5 | France | Humain | 0,50 | Sensible | |
| | FHM-PA6 | France | Humain | 0,38 | Sensible | |
| *Shewanella xianemensis* | 111P | France | Humain | 0,125 | Sensible | |
| | 111A | France | Humain | 0,094 | Sensible | |
| *Acinetobacter nosocomialis* | ABG13S | France | Humain | 0,064 | Sensible | |
| *Acinetobacter pitti* | MK | France | Humain | 0,125 | Sensible | |

| **Gram-positifs résistants à la colistine** | | | | | | |
|---|---|---|---|---|---|---|
| *Enterococcus faecium* | DSM17050 | Allemagne | Humain | >256 | *vanA* | |
| | DSM13590 | Allemagne | Humain | >256 | *vanA* | |
| | DSM25698 | Corée du Sud | Humain | >256 | *vanA* | |
| | DSM25697 | Corée du Sud | Humain | >256 | *vanA* | |
| | FHMVRE1 | France | Humain | >256 | Sensible | De cette étude |
| | FHMVRE2 | France | Humain | >256 | Inconnu | De cette étude |
| | VRE | France | Humain | 24 | Inconnu | Non publié |
| *Enterococcus faecalis* | JH2-2: Tn 1549 | France | Humain | 64 | *vanB* | (Launay et al., 2006) |
| *Weissela cibaria* | P18A | Laos | Cochon | >256 | Inconnu | De cette étude |
| *Weissela confusa* | P18B | Laos | Cochon | >256 | Inconnu | De cette étude |
| *Leuconostoc lactis* | P18C | Laos | Cochon | >256 | Inconnu | De cette étude |

| **Gram-positifs sensibles à la vancomycine** | | | | | | |
|---|---|---|---|---|---|---|
| *Staphylococcus aureus* | CF_Marseille | France | Humain | 1 | Sensible | (Rolain et al., 2009) |
| | FHM-SA | France | Humain | NC | Sensible | Non publié |
| *Enterococcus faecium* | TH26 | Thaïlande | Humain | 0,75 | Sensible | De cette étude |
| | 349 | Algérie | Poulet | 0,75 | Sensible | De cette étude |
| | TH12 | Thaïlande | Humain | 2 | Sensible | De cette étude |
| | LH165 | Laos | Humain | 0,75 | Sensible | De cette étude |
| | A17 | Algérie | Poulet | 0,75 | Sensible | De cette étude |
| | TH43 | Thaïlande | Humain | 0,75 | Sensible | De cette étude |
| | TH95 | Thaïlande | Humain | 0,75 | Sensible | De cette étude |
| | 282 | Algérie | Poulet | 0,75 | Sensible | De cette étude |
| *Enterococcus faecalis* | JH2-2 : C2 | Thaïlande | Humain | 1,5 | Sensible | De cette étude |
| | JH2-2S | Thaïlande | Humain | 1,5 | Sensible | De cette étude |
| *Enterococcus gallinarum* | SE3 | Algérie | Poulet | 1,5 | Sensible | De cette étude |
| *Enterococcus casseliflavus* | SE3 | Algérie | Poulet | 1,5 | Sensible | De cette étude |
| *Enterococcus hirae* | LH111 | Laos | Humain | 1 | Sensible | De cette étude |

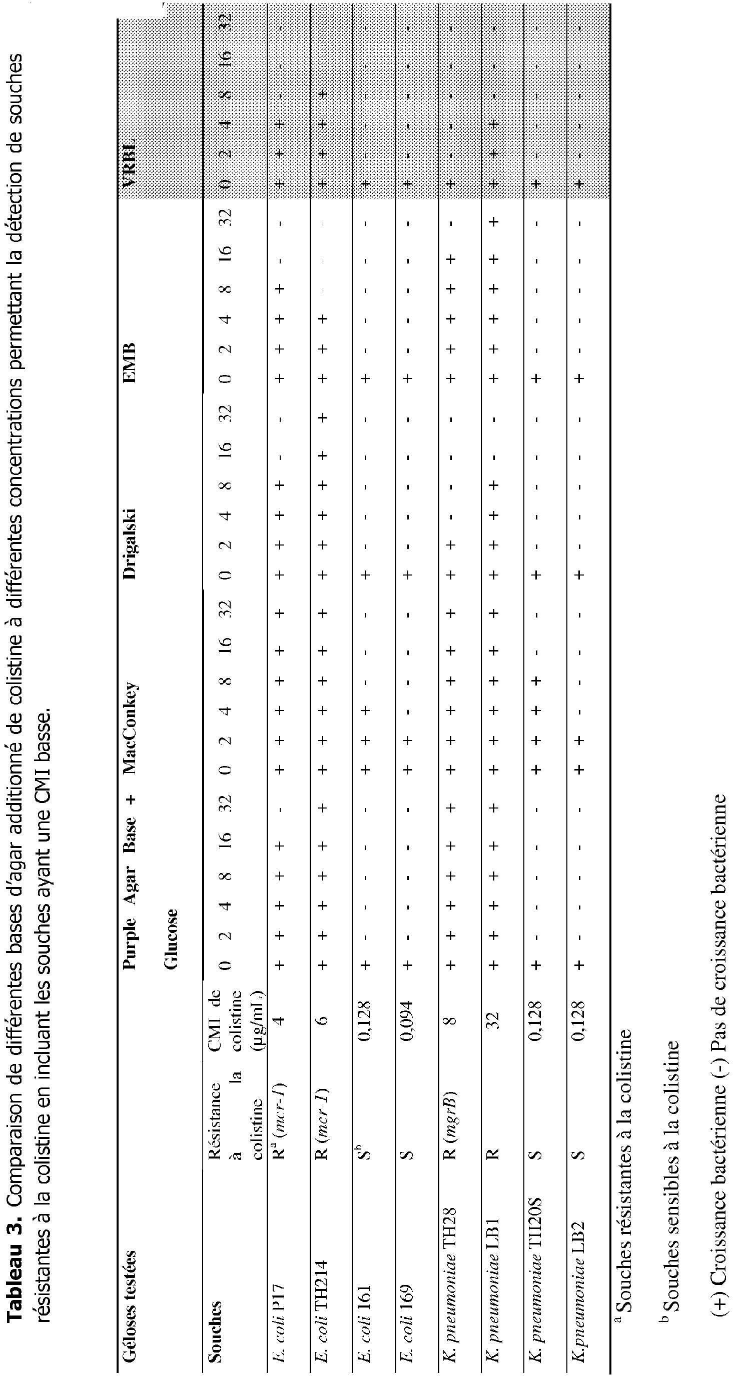

Références bibliographiques
1. Olaitan AO, Diene SM, Kempf M, Berrazeg M, Bakour S, Gupta SK, Thongmalayvong B, Akkhavong K, Somphavong S, Paboriboune P, Chaisiri K, Komalamisra C, Adelowo OO, Fagade OE, Banjo OA, Oke AJ, Adler A, Assous MV, Morand S, Raoult D, Rolain J-M. 2014. Worldwide emergence of colistin resistance in Klebsiella pneumoniae from healthy humans and patients in Lao PDR, Thailand, Israel, Nigeria and France owing to inactivation of the PhoP/PhoQ regulator mgrB: an epidemiological and molecular study. Int J Antimicrob Agents 44:500-7.
2. Kempf I, Fleury MA, Drider D, Bruneau M, Sanders P, Chauvin C, Madec J-Y, Jouy E. 2013. What do we know about resistance to colistin in Enterobacteriaceae in avian and pig production in Europe? Int J Antimicrob Agents 42:379-383.
3. Biswas S, Brunel J-M, Dubus J-C, Reynaud-Gaubert M, Rolain J-M. 2012. Colistin: an update on the antibiotic of the 21st century. Expert Rev Anti Infect Ther 10:917-934.
4. Cai Y, Lee W, Kwa AL. 2015. Polymyxin B versus colistin: an update. Expert Rev Anti Infect Ther 13:1-17.
5. Olaitan AO, Morand S, Rolain J-M. 2016. Emergence of colistin-resistant bacteria in humans without colistin usage: a new worry and cause for vigilance. Int J Antimicrob Agents 47:1-3.
6. Liu Y-Y, Wang Y, Walsh TR, Yi L-X, Zhang R, Spencer J, Doi Y, Tian G, Dong B, Huang X, Yu L-F, Gu D, Ren H, Chen X, Lv L, He D, Zhou H, Liang Z, Liu J-H, Shen J. 2016. Emergence of plasmid-mediated colistin resistance mechanism MCR-1 in animais and human beings in China: a microbiological and molecular biological study. Lancet Infect Dis 16:161-8.
7. Olaitan AO, Morand S, Rolain J-M. 2014. Mechanisms of polymyxin résistance: acquired and intrinsic resistance in bacteria. Front Microbiol 5:643.
8. Baron S, Hadjadj L, Rolain JM, Olaitan AO. 2016. Molecular mechanisms of polymyxin resistance: Knowns and unknowns. Int J Antimicrob Agents 48:583-591.
9. Xavier BB, Lammens C, Ruhal R, Kumar-Singh S, Butaye P, Goossens H M-KS. 2016. Identification of a novel plasmid-mediated colistin-resistance gene, mcr-2, in Escherichia coli, Belgium, June 2016. Eurosurveillance 21.
10. Zeng K-J, Doi Y, Patil S, Huang X, Tian G-B. 2016. Emergence of plasmid-mediated mcr-1 gene in colistin-resistant Enterobacter aerogenes and Enterobacter cloacae. Antimicrob Agents Chemother.
11. Cannatelli A, Giani T, Antonelli A, Principe L, Luzzaro F, Rossolini GM. 2016. First detection of the mcr-1 colistin resistance gene in Escherichia coli, Italy. Antimicrob Agents Chemother.
12. Quesada A, Ugarte-Ruiz M, Iglesias MR, Porrero MC, Martinez R, Florez-Cuadrado D, Campos MJ, García M, Píriz S, Sáez JL, Domínguez L. 2016. Detection of plasmid mediated colistin resistance (MCR-1) in Escherichia coli and Salmonella enterica isolated from poultry and swine in Spain. Res Vet Sci 105:134-5.
13. Skov RL, Monnet DL. 2016. Plasmid-mediated colistin resistance (mcr-1 gene): three months later, the story unfolds. Euro Surveill Bull Eur sur les Mal Transm = Eur Commun Dis Bull 21.
14. Rolain J-M, Olaitan AO. 2016. Plasmid-mediated colistin resistance: the final blow to colistin? Int J Antimicrob Agents 47:4-5.
15. Chabou S, Leangapichart T, Okdah L, Le Page S, Hadjadj L, Rolain J-M. 2016. Real-time quantitative PCR assay with Taqman* probe for rapid detection of MCR-1 plasmid-mediated colistin resistance. New Microbes New Infect.
16. Jayol A, Dubois V, Poirel L, Nordmann P. 2016. Rapid detection of polymyxin-resistant Enterobacteriaceae from blood cultures. J Clin Microbiol.
17. Nordmann P, Jayol A, Poirel L. 2016. Rapid detection of polymyxin resistance in Enterobacteriaceae. Emerg Infect Dis 22:1038-1043.
18. Nordmann P, Jayol A, Poirel L. 2016. A universal culture medium for screening polymyxin-resistant gram negatives. J Clin Microbiol.
19. Olaitan AO, Dia NM, Gautret P, Benkouiten S, Belhouchat K, Drali T, Parola P, Brouqui P, Memish Z, Raoult D, Rolain J-M. 2015. Acquisition of extended-spectrum cephalosporin- and colistin-resistant Salmonella enterica subsp. enterica serotype Newport by pilgrims during Hajj. Int J Antimicrob Agents 45:600-4.
20. Olaitan AO, Rolain J-M. 2015. Interruption of mgrB in the mediation of colistin resistance in Klebsiella oxytoca. Int J Antimicrob Agents 46:354-5.
21. Diene SM, Merhej V, Henry M, El Filali A, Roux V, Robert C, Azza S, Gavory F, Barbe V, La Scola B, Raoult D, Rolain J-M. 2013. The rhizome of the multidrug-resistant Enterobacter aerogenes genome reveals how new "killer bugs" are created because of a sympatric lifestyle. Mol Biol Evol 30:369-83.
22. Rolain J-M, Kempf M, Leangapichart T, Chabou S, Olaitan O, Le Page S, Morand S, Raoult D. 2016. Title: Plasmid-mediated mcr-1 gene in colistin-resistant clinical isolates of Klebsiella 1 pneumoniae in France and Laos. 2.
23. Berrazeg M, Hadjadj 1 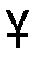 L, Ayad A, Drissi M, Rolain JM. 2016. First detected human case in Algeria of mcr-1 plasmid mediated colistin resistance: a Conflict of interest: none to declare 1-7.
24. Olaitan AO, Chabou S, Okdah L, Morand S, Rolain J-M. 2016. Dissemination of the mcr-1 colistin resistance gene. Lancet Infect Dis 16:147.
25. Leangapichart T, Gautret P, Brouqui P, Mimish Z, Raoult D, Rolain J-M. 2016. Acquisition of mcr-1 plasmid-mediated colistin resistance in Escherichia coli and Klebsiella pneumoniae during Hajj 2013 and 2014. Antimicrob Agents Chemother 60:6998-7000.
26. Launay A, Ballard SA, Johnson PDR, Grayson ML, Lambert T. 2006. Transfer of Vancomycin Resistance Transposon Tn 1549 from Clostridium symbiosum to Enterococcus spp. in the Gut of Gnotobiotic Mice 50:1054-1062.
27. Rolain J-M, François P, Hernandez D, Bittar F, Richet H, Fournous G, Mattenberger Y, Bosdure E, Stremler N, Dubus J-C, Sarles J, Reynaud-Gaubert M, Boniface S, Schrenzel J, Raoult D. 2009. Genomic analysis of an emerging multiresistant Staphylococcus aureus strain rapidly spreading in cystic fibrosis patients revealed the presence of an antibiotic inducible bacteriophage. Biol Direct 4:1.
28. Domingo MC, Huletsky A, Giroux R, Boissinot K, Picard FJ, Lebel P, Ferraro MJ, Bergeron MG. 2005. High prevalence of glycopeptide resistance genes vanB, vanD, and vanG not associated with enterococci in human fecal flora. Antimicrob Agents Chemother 49:4784-4786.

## Revendications

1. Milieu de culture solide sélectif polyvalent pour isoler et sélectionner, à partir d'échantillons en contenant, des bactéries choisies parmi (a) les bactéries à Gram-négatif fermentantes et non fermentantes résistantes à la colistine et (b) les bactéries à Gram-positif résistantes à la vancomycine, **caractérisé en ce qu'**il est constitué essentiellement d'une gélose d'agar additionnée d'un agent chromogène consistant dans du pourpre de bromocrésol, un sucre et de deux composés antibiotiques consistant dans la vancomycine et la colistine.

2. Milieu de culture selon la revendication 1 **caractérisé en ce que** le dit sucre est du glucose.

3. Milieu de culture selon la revendication 1 ou 2 **caractérisé en ce qu'**il comprend dans de l'eau des concentrations de:
- 2 à 10% en poids en agar contenant de 0.01 à 0.1% de dit agent chromogène pourpre de bromocrésol,
- 0.1. à 10% en poids de sucre, de préférence de glucose,
- 2 à 8 µg/mL de sulfate de colistine, et
- 25. à 100 µg/mL de vancomycine.

4. Milieu de culture selon la revendication 3 **caractérisé en ce qu'**il comprend :
- 3.1% en poids en agar contenant de 0.065% de pourpre de bromocrésol,
- 0.75% de glucose,
- 4.µg/mL sulfate de colistine, et
- 50.µg/mL de vancomycine.

5. Méthode d'isolement et sélection de bactéries choisies parmi (a) les bactéries à Gram-négatif résistantes à la colistine, (b) les bactéries à Gram-positif résistantes à la vancomycine, **caractérisé en ce qu'**on réalise une culture d'un d'échantillon de bactéries en contenant, de préférence un échantillon de prélèvement clinique, à l'aide d'un milieu de culture sélectif polyvalent solide selon l'une des revendications 1 à 4.

6. Méthode selon la revendication 5 **caractérisé en ce que** les dites bactéries à Gram-négatif résistantes à la colistine sont des Entérobactéries.

7. Méthode selon la revendication 6 **caractérisé en ce que** les dites Entérobactéries pathogènes sont choisies parmi les bactéries suivantes *Escherichia, Shigella, Salmonella, Klebsiella, Enterobacter, Providencia, Morganella, Citrobacter, Hafnia* et *Yersinia.*

8. Méthode selon la revendication 7 **caractérisé en ce que** les dites bactéries à Gram-négatif résistantes à la colistine comprennent *Escherichia coli, Klebsiella pneumoniae, Klebsiella oxytoca, Enterobacter aerogenes, Enterobacter asburiae, Enterobacter cloacae, Salmonella enterica, Proteus mirabilis, Proteus vulgaris, Providencia alcalifaciens, Providencia rettgeri, Morganella morganii* et *Serratia marcescens.*

9. Méthode selon la revendication 8 **caractérisé en ce que** les dites bactéries à Gram-négatif résistantes à la colistine comprennent Escherichia coli.

10. Méthode selon la revendication 5 **caractérisé en ce que** les dites bactéries à Gram-négatif résistantes à la colistine sont des bactéries présentes dans les prélèvements cliniques, de préférence des prélèvements pulmonaires, de patients atteints par la mucoviscidose.

11. Méthode selon la revendication 10 **caractérisé en ce que** les dites bactéries présentes dans les prélèvements cliniques des patients atteints par la mucoviscidose sont choisies parmi :
- les bactéries naturellement résistantes à la colistine suivantes : *Burkholderia cepacia, Chryseobacterium gleum, Chryseobacterium oranimense, Chryseobacterium indologenes, Inquilinus limosus, Pandoraea pulmonicola, Brevundimonas diminuta, Shewanella xianemensis, Shewanella putrefaciens* et *Ochrobactrum anthropi* ; et
- les bactéries ayant une résistance acquise à la colistine suivantes : *Acinetobacter pitti, Acinetobacter nosocomialis Achromobacter xylosoxidans, Stenotrophomonas maltophilia Pseudomonas aeruginosa* et *Acinetobacter baumannii.*

12. Méthode selon l'une des revendications 5 à 11 **caractérisé en ce que** les dites bactéries à Gram-négatif résistantes à la colistine sont choisies parmi (il) des bactéries naturellement résistantes à la Colistine, (i2) des bactéries résistantes à la colistine de façon acquise par mutation chromosomique spontanée et (i3) des bactéries résistantes à la colistine par l'acquisition d'un plasmide de résistance contenant le gène mcr-1.

13. Méthode selon la revendication 12 **caractérisé en ce que** les dites bactéries à Gram-négatif résistantes à la colistine sont choisies parmi des bactéries résistantes à la Colistine par l'acquisition d'un plasmide de résistance contenant le gène mcr-1.

14. Méthode selon la revendication 5 **caractérisé en ce que** les dites bactéries sont des bactéries à Gram positif résistantes à la vancomycine choisies parmi :
- les bactéries ayant une résistance acquise à la vancomycine des genres Staphylococcus et Enterococcus, et
- les bactéries naturellement résistantes à la vancomycine des genres *Pediococcus, Weissela, Lactobacillus* et *Leuconostoc.*

15. Méthode selon la revendication 14 **caractérisé en ce que** les dites bactéries à Gram positif résistantes à la vancomycine sont choisies parmi les bactéries suivantes : *Staphylococcus aureus, Enterococcus faecium, Enterococcus faecalis, Enterococcus gallinarum, Weissela cibaria* et *Weissela confusa.*

16. Méthode selon la revendication 15 **caractérisé en ce que** les dites bactéries à Gram-positif résistantes à la vancomycine sont des Entérocoques.

17. Méthode selon l'une des revendications 5 à 11 **caractérisé en ce qu'**on réalise une culture d'échantillon de prélèvement clinique, de préférence de selles.

## Patentansprüche

1. Selektives festes Mehrzweck-Kulturmedium zum Isolieren und Auswählen von Bakterien, ausgewählt aus (a) fermentierenden und nicht fermentierenden Gram-negativen Bakterien, die gegen Colistin resistent sind, und (b) Gram-positiven Bakterien, die gegen Vancomycin resistent sind, aus Proben, die diese enthalten, **dadurch gekennzeichnet, dass** es im Wesentlichen aus einem Agar-Agar besteht, dem ein chromogenes Mittel zugesetzt ist, das aus Bromkresolpurpur, einem Zucker und zwei antibiotischen Verbindungen besteht, die aus Vancomycin und Colistin bestehen.

2. Kulturmedium nach Anspruch 1, **dadurch gekennzeichnet, dass** der Zucker Glucose ist.

3. Kulturmedium nach Anspruch 1 oder 2, **dadurch gekennzeichnet, dass** es in Wasser Konzentrationen umfasst von:
- 2 bis 10 Gew.-% Agar, enthaltend 0,01 bis 0,1 % chromogenes Bromkresolpurpurmittel,
- 0,1. bis 10 Gew.-% Zucker, vorzugsweise Glucose,
- 2 bis 8 µg/mL Colistinsulfat und
- 25 bis 100 µg/mL Vancomycin.

4. Kulturmedium nach Anspruch 3, **dadurch gekennzeichnet, dass** es umfasst:
- 3,1 Gew.-% Agar, enthaltend 0,065 % Bromkresolpurpur,
- 0,75 % Glucose,
- 4 µg/mL Colistinsulfat und
- 50 µg/mL Vancomycin.

5. Verfahren zum Isolieren und Auswählen von Bakterien, ausgewählt aus (a) Colistin- resistenten Gram-negativen Bakterien, (b) Vancomycin-resistenten Gram-positiven Bakterien, **dadurch gekennzeichnet, dass** eine Kultur einer Probe von Bakterien, die diese enthält, vorzugsweise einer klinischen Entnahmeprobe, mit Hilfe eines festen selektiven Mehrzweck-Kulturmediums nach einem der Ansprüche 1 bis 4 hergestellt wird.

6. Verfahren nach Anspruch 5, **dadurch gekennzeichnet, dass** die Colistin-resistenten Gram-negativen Bakterien Enterobacteriaceae sind.

7. Verfahren nach Anspruch 6, **dadurch gekennzeichnet, dass** die pathogenen Enterobacteriaceae ausgewählt sind aus den folgenden Bakterien: *Escherichia, Shigella, Salmonella, Klebsiella, Enterobacter, Providencia, Morganella, Citrobacter, Hafnia* und *Yersinia.*

8. Verfahren nach Anspruch 7, **dadurch gekennzeichnet, dass** die Colistin-resistenten Gram-negativen Bakterien *Escherichia coli, Klebsiella pneumoniae, Klebsiella oxytoca, Enterobacter aerogenes, Enterobacter asburiae, Enterobacter cloacae, Salmonella enterica, Proteus mirabilis, Proteus vulgaris, Providencia alcalifaciens, Providencia rettgeri, Morganella morganii* und *Serratia marcescens* umfassen.

9. Verfahren nach Anspruch 8, **dadurch gekennzeichnet, dass** die Colistin-resistenten Gram-negativen Bakterien Escherichia coli umfassen.

10. Verfahren nach Anspruch 5, **dadurch gekennzeichnet, dass** die Colistin-resistenten Gram-negativen Bakterien Bakterien sind, die in klinischen Proben, vorzugsweise Lungenproben, von an Mukoviszidose erkrankten Patienten vorhanden sind.

11. Verfahren nach Anspruch 10, **dadurch gekennzeichnet, dass** die in den klinischen Proben vorhandenen Bakterien der an Mukoviszidose erkrankten Patienten ausgewählt sind aus:
- den folgenden Bakterien, die von Natur aus gegen Colistin resistent sind: *Burkholderia cepacia, Chryseobacterium gleum, Chryseobacterium oranimense, Chryseobacterium indologenes, Inquilinus limosus, Pandoraea pulmonicola, Brevundimonas diminuta, Shewanella xianemensis, Shewanella putrefaciens* und *Ochrobactrum anthropi*; und
- den folgenden Bakterien, die eine erworbene Resistenz gegen Colistin aufweisen: *Acinetobacter pitti, Acinetobacter nosocomialis Achromobacter xylosoxidans, Stenotrophomonas maltophilia Pseudomonas aeruginosa* und *Acinetobacter baumannii.*

12. Verfahren nach einem der Ansprüche 5 bis 11, **dadurch gekennzeichnet ist, dass** die Colistin-resistenten Gram-negativen Bakterien ausgewählt sind aus (i1) Bakterien, die von Natur aus gegen Colistin resistent sind, (i2) Bakterien, die auf durch spontane chromosomale Mutation erworbene Weise gegen Colistin resistent sind, und (i3) Bakterien, die durch den Erwerb eines Resistenzplasmids, das das mcr-1-Gen enthält, gegen Colistin resistent sind.

13. Verfahren nach Anspruch 12, **dadurch gekennzeichnet, dass** die Colistin-resistenten Gram-negativen Bakterien aus Bakterien ausgewählt sind, die durch den Erwerb eines das mcr-1-Gen enthaltenden Resistenzplasmids gegen Colistin resistent sind.

14. Verfahren nach Anspruch 5, **dadurch gekennzeichnet, dass** die Bakterien Gram-positive Bakterien sind, die gegen Vancomycin resistent sind, welche ausgewählt sind aus:
- den Bakterien mit einer erworbenen Vancomycin-Resistenz der Gattungen Staphylococcus und Enterococcus, und
- den von Natur aus gegen Vancomycin resistenten Bakterien der Gattungen *Pediococcus, Weissela, Lactobacillus* und *Leuconostoc.*

15. Verfahren nach Anspruch 14, **dadurch gekennzeichnet, dass** die Vancomycin-resistenten Gram-positiven Bakterien aus den folgenden Bakterien ausgewählt sind: *Staphylococcus aureus, Enterococcus faecium, Enterococcus faecalis, Enterococcus gallinarum, Weissela cibaria* und *Weissela confusa.*

16. Verfahren nach Anspruch 15, **dadurch gekennzeichnet, dass** die Vancomycin-resistenten Gram-positiven Bakterien Enterokokken sind.

17. Verfahren nach einem der Ansprüche 5 bis 11, **dadurch gekennzeichnet, dass** eine Kultur einer klinischen Entnahmeprobe, vorzugsweise von Stuhl, hergestellt wird.

## Claims

1. A multi-purpose selective solid culture medium for isolating and selecting, from samples containing it, bacteria selected from (a) colistin-resistant fermenting and non-fermenting Gram-negative bacteria and (b) vancomycin-resistant Gram-positive bacteria, **characterized in that** it consists essentially of an agar-agar to which is added a chromogenic agent consisting of bromocresol purple, a sugar and two antibiotic compounds consisting of vancomycin and colistin.

2. The culture medium as claimed in claim 1 **characterized in that** said sugar is glucose.

3. The culture medium as claimed in claim 1 or 2 **characterized in that** it comprises in water concentrations of:
- 2 to 10% by weight agar containing from 0.01 to 0.1% of said bromocresol purple chromogenic agent,
- 0.1. to 10% by weight sugar, preferably glucose,
- 2 to 8 µg/mL colistin sulfate, and
- 25 to 100 µg/mL vancomycin.

4. The culture medium as claimed in claim 3 **characterized in that** it comprises:
- 3.1% by weight agar containing 0.065% bromocresol purple,
- 0.75% glucose,
- 4 µg/mL colistin sulfate, and
- 50 µg/mL vancomycin.

5. A method for isolating and selecting bacteria chosen from (a) colistin-resistant Gram-negative bacteria, (b) vancomycin-resistant Gram-positive bacteria, **characterized in that** a sample of bacteria containing it, preferably a clinical specimen, is cultured using a solid multi-purpose selective culture medium according to one of claims 1 to 4.

6. The method as claimed in claim 5 **characterized in that** said colistin-resistant Gram-negative bacteria are Enterobacteriaceae.

7. The method as claimed in claim 6 **characterized in that** said pathogenic Enterobacteriaceae are selected from the following bacteria: *Escherichia, Shigella, Salmonella, Klebsiella, Enterobacter, Providencia, Morganella, Citrobacter, Hafnia* and *Yersinia.*

8. The method as claimed in claim 7 **characterized in that** said colistin-resistant Gram-negative bacteria include *Escherichia coli, Klebsiella pneumoniae, Klebsiella oxytoca, Enterobacter aerogenes, Enterobacter asburiae, Enterobacter cloacae, Salmonella enterica, Proteus mirabilis, Proteus vulgaris, Providencia alcalifaciens, Providencia rettgeri, Morganella morganii* and *Serratia marcescens.*

9. The method as claimed in claim 8 **characterized in that** said colistin-resistant Gram-negative bacteria include *Escherichia coli.*

10. The method as claimed in claim 5 **characterized in that** said colistin-resistant Gram-negative bacteria are bacteria present in clinical specimens, preferably lung specimens, from cystic fibrosis patients.

11. The method as claimed in claim 10 **characterized in that** said bacteria present in the clinical specimens of cystic fibrosis patients are selected from:
- the following naturally colistin-resistant bacteria: *Burkholderia cepacia, Chryseobacterium gleum, Chryseobacterium oranimense, Chryseobacterium indologenes, Inquilinus limosus, Pandoraea pulmonicola, Brevundimonas diminuta, Shewanella xianemensis, Shewanella putrefaciens* and *Ochrobactrum anthropi*; and
- the following bacteria with acquired colistin resistance: *Acinetobacter pitti, Acinetobacter nosocomialis, Achromobacter xylosoxidans, Stenotrophomonas maltophilia, Pseudomonas aeruginosa* and *Acinetobacter baumannii.*

12. The method as claimed in one of claims 5 to 11 **characterized in that** said colistin-resistant Gram-negative bacteria are selected from (i1) naturally colistin-resistant bacteria, (i2) bacteria resistant to colistin acquired by spontaneous chromosomal mutation and (i3) bacteria resistant to colistin by the acquisition of a resistance plasmid containing the mcr-1 gene.

13. The method as claimed in claim 12 **characterized in that** said colistin-resistant Gram-negative bacteria are selected from bacteria resistant to colistin by the acquisition of a resistance plasmid containing the mcr-1 gene.

14. The method as claimed in claim 5 **characterized in that** said bacteria are vancomycin-resistant Gram-positive bacteria selected from:
- bacteria having acquired resistance to vancomycin of the genera *Staphylococcus* and *Enterococcus,* and
- naturally vancomycin-resistant bacteria of the genera *Pediococcus, Weissela, Lactobacillus* and *Leuconostoc.*

15. The method as claimed in claim 14 **characterized in that** said vancomycin-resistant Gram-positive bacteria are selected from the following bacteria: *Staphylococcus aureus, Enterococcus faecium, Enterococcus faecalis, Enterococcus gallinarum, Weissela cibaria* and *Weissela confusa.*

16. The method as claimed in claim 15 **characterized in that** said vancomycin-resistant Gram-positive bacteria are Enterococcaceae.

17. The method as claimed in one of claims 5 to 11 **characterized in that** a clinical specimen, preferably stool, is cultured.
